# EUROPEAN PATENT SPECIFICATION

(11) **EP 4 531 773 B1**
(45) Date of publication and mention of the grant of the patent: **04.11.2026**
(21) Application number: 23730585.9
(22) Date of filing: 15.05.2023
(51) Int. Cl.: A61F 9/00, A61B 17/34, A61M 5/32

(54) **OCULAR NEEDLE GUIDE**
AUGENNADELFÜHRUNG
GUIDE D'AIGUILLE OCULAIRE

(30) Priority: 24.05.2022 US 202263345117 P
(43) Date of publication of application: 09.04.2025
(73) Proprietor: Janssen Biotech, Inc., Horsham, PA 19044 (US)
(72) Inventor: FLEMING, James, Piscataway, New Jersey 08854 (US); JAIN, Sanjay, Malvern, Pennsylvania 19355 (US); CEDRO, Rudolph, Raritan, New Jersey 08869 (US); KENNEDY, Eric Steven, Cincinnati, Ohio 45241 (US); CRAFT, Travis Michael, Cincinnati, Ohio 45241 (US); KOENIG, Abigail, Cincinnati, Ohio 45241 (US)
(74) Representative: Carpmaels & Ransford LLP
(86) International application number: PCT/IB2023/054991
(87) International publication number: WO 2023/228002

(56) References cited:
- EP-A1- 2 265 229
- EP-A1- 3 730 102
- WO-A1-2016/196841

## Description

### TECHNICAL FIELD

This disclosure relates generally to pharmaceutical devices and related methods for delivering a therapeutic agent, and more particularly to ocular guides and related methods for facilitating needle access to an interior of an eye.

### BACKGROUND

The eye is a complex organ with a variety of specialized tissues that provide the optical and neurological processes for vision. Accessing the eye for medical treatment is hindered by the small size and delicate nature of the tissues. The posterior region of the eye, including the retina, macula, choroid, and optic nerve, is especially difficult to access due to the recessed position of the eye within the orbital cavity. In addition, topical eye drops penetrate poorly into the posterior region, further restricting treatment options.

The suprachoroidal and subchoroidal spaces are potential spaces in the eye for treatment options. The suprachoroidal space is located between the choroid, which is the inner vascular tunic, and the sclera, the outer layer of the eye. The subchoroidal space is located below the choroid and above the retina. The choroidal spaces extend from the anterior portion of the eye near the ciliary body to the posterior end of the eye near the optic nerve. Normally the suprachoroidal space is not evident due to the close apposition of the choroid to the sclera from the intraocular pressure of the eye. Since there is no substantial attachment of the choroid to the sclera, the tissues separate to form the suprachoroidal space when fluid accumulation or other conditions occur. The suprachoroidal space provides a potential route of access from the anterior region of the eye to treat the posterior region.

Accessing the suprachoroidal or subchoroidal space of the eye is a delicate procedure that requires substantial skill and time. There are several methods for accessing this space in order to deliver a therapeutic agent, but the complexity and time required are a barrier to widespread use. Any attempt to puncture the sclera with a needle in an attempt to access a specific depth (and therefore a specific inter-layer space) faces the challenge of providing enough force to penetrate the relatively tough sclera tissue without over penetrating. This challenge is exaggerated by the compliant nature of the eye and no means to provide support to the opposite side of the tissue being penetrated.
WO 2016/196841 A1 discloses that placing an intraocular shunt *ab externo* into an eye can include inserting the shunt into the eye and either before and/or after insertion, ballooning a target outflow region of the eye to permit an outflow end of the shunt to be enveloped within the ballooned target outflow region. An injector docking device can optionally be used to guide insertion of the needle and shunt into the eye.
EP 2 265 229 A1 discloses an injection device for injecting an agent into an eye that comprises: an injection needle and a substrate whereon the injection needle is attached, the device being characterized in that it comprises a locating mark disposed such that the same can be placed in contact with a bearing region determined by the eye surface before any contact by the injection needle with said surface, and then can be kept in contact with said bearing region during a stage wherein said injection needle penetrates through said eye surface.
The foregoing background discussion is intended solely to aid the reader. It is not intended to limit the innovations described herein. Thus, the foregoing discussion should not be taken to indicate that any particular element of a prior system is unsuitable for use with the innovations described herein, nor is it intended to indicate that any element is essential in implementing the innovations described herein.

### SUMMARY

The invention is defined by the appended independent claims. Further embodiments are defined in the dependent claims.

This summary is provided to introduce a selection of concepts in a simplified form that are further described below in the Detailed Description section. This Summary is not intended to identify key features or essential features of the claimed subject matter, nor is it intended to be used to limit the scope of the claimed subject matter. Furthermore, the claimed subject matter is not constrained to limitations that solve any or all disadvantages noted in any part of this disclosure.

### BRIEF DESCRIPTION OF THE DRAWINGS

The foregoing summary, as well as the following detailed description of illustrative embodiments of the present application, will be better understood when read in conjunction with the appended drawings. For the purposes of illustrating the present application, there are shown in the drawings illustrative embodiments of the disclosure. It should be understood, however, that the application is not limited to the precise arrangements and instrumentalities shown. In the drawings:
FIG. 1 illustrates a side view of an ocular needle guide positioned on an eye, according to an aspect of this disclosure.
FIG. 2 illustrates cross-sectional view of the ocular needle guide in a first orientation positioned on the eye taken along line 2-2 shown in FIG. 1.
FIG. 3 illustrates cross-sectional view of the ocular needle guide in a second orientation positioned on the eye taken along line 2-2 shown in FIG. 1.
FIG. 4 illustrates a close-up view of the ocular needle guide shown in FIG. 2.
FIG. 5 illustrates a cross-sectional view of an ocular needle guide in a first orientation positioned on an eye, according to another aspect of this disclosure.
FIG. 6 illustrates cross-sectional view of the ocular needle guide illustrated in FIG. 5 in a second orientation positioned on the eye.
FIG. 7 illustrates a close-up view of the ocular needle guide shown in FIG. 5.
FIG. 8 illustrates a cross-sectional view of an ocular needle guide in a first orientation, according to another aspect of this disclosure.
FIG. 9 illustrates a cross-sectional view of the ocular needle guide illustrated in FIG. 8 in a second orientation.
FIG. 10 illustrates a cross-sectional view of an ocular needle guide positioned on an eye, according to another aspect of this disclosure.
FIG. 11 illustrates a top perspective view of the ocular needle guide shown in FIG. 10.
FIG. 12 illustrates a bottom view of the ocular needle guide shown in FIG. 10.
FIG. 13 illustrates a cross-sectional view of an ocular needle guide in a first orientation positioned on an eye, according to another aspect of this disclosure.
FIG. 14 illustrates a cross-sectional view of the ocular needle guide illustrated in FIG. 13 in a second orientation.
FIG. 15 illustrates a top perspective view of the ocular needle guide shown in FIG. 13.
FIG. 16 illustrates a bottom view of the ocular needle guide shown in FIG. 13.
FIG. 17 illustrates a cross-sectional view of an ocular needle guide positioned on an eye, according to another aspect of this disclosure.
FIG. 18 illustrates a cross-sectional view of an ocular needle guide positioned on an eye, according to another aspect of this disclosure.
FIG. 19 illustrates a top perspective view of the ocular needle guide shown in FIG. 18.
FIG. 20 illustrates a bottom view of the ocular needle guide shown in FIG. 18.
FIG. 21 illustrates a cross-sectional view of an ocular needle guide positioned on an eye, according to another aspect of this disclosure.
FIG. 22 illustrates a top perspective view of the ocular needle guide shown in FIG. 21.
FIG. 23 illustrates a cross-sectional view of an ocular needle guide positioned on an eye, according to another aspect of this disclosure.
FIGS. 24A-24F illustrate top perspective views of ocular needle guides, according to other aspects of this disclosure.
FIG. 25 illustrates a cross-sectional view of an ocular needle guide in a first orientation positioned on an eye, according to another aspect of this disclosure.
FIG. 26 illustrates a cross-sectional view of the ocular needle guide illustrated in FIG. 25 in a second orientation.

### DETAILED DESCRIPTION

Certain terminology used in this description is for convenience only and is not limiting. The words "axial", "radial", "circumferential", "outward", "inward", "upper,", "lower", "top", and "bottom" designate directions in the drawings to which reference is made. As used herein, the term "substantially," "approximately," derivatives thereof, and words of similar import, when used to describe a size, shape, orientation, distance, spatial relationship, or other parameter includes the stated size, shape, orientation, distance, spatial relationship, or other parameter, and can also include a range up to 10% more and up to 10% less than the stated parameter, including 5% more and 5% less, including 3% more and 3% less, including 1% more and 1% less. The term "substantially," "approximately," and the like are intended to mean considerable in extent or largely but not necessarily wholly (but can include wholly) that which is specified. All ranges disclosed herein are inclusive of the recited endpoint and independently combinable (for example, the range of "from 2 grams to 10 grams" is inclusive of the endpoints, 2 grams and 10 grams, and all the intermediate values). The terminology includes the above-listed words, derivatives thereof and words of similar import.

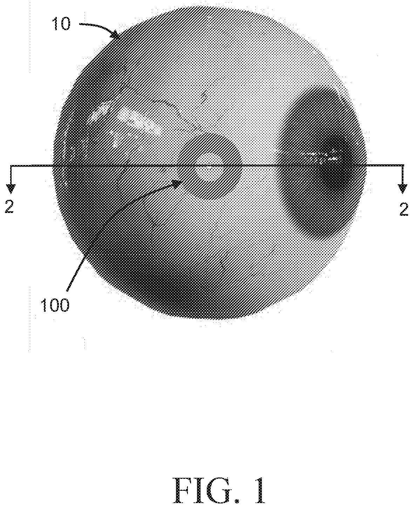

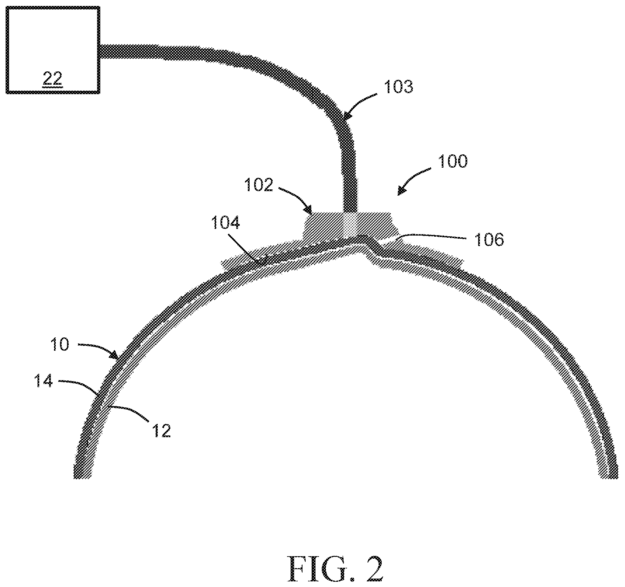

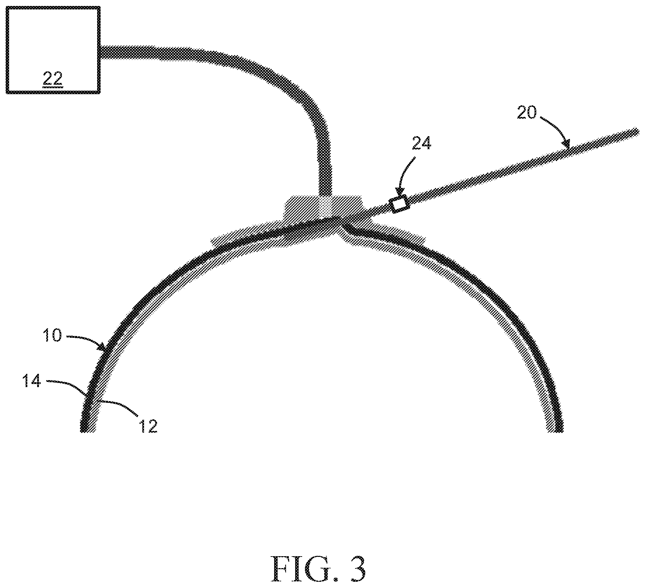

FIG. 1 illustrates an ocular needle guide 100 positioned on an eye 10, and FIGS. 2 and 3 illustrate cross-sectional views of the ocular needle guide 100 positioned on the eye 10 taken along line 2-2 shown in FIG. 1. The eye 10 has a spherical shape and layers with physical properties that present challenges when trying to access a space, for example, either above or below a choroid 12 layer of the eye 10. The ocular needle guides described herein employ a shaped surface to temporarily remodel the shape of the eye 10 to align the underlying target space below a sclera 14 of the eye 10 (e.g. sub- or supra- choroidal space) with a needle 20.

The ocular needle guide 100 includes a housing 102. The housing 102 is shaped to be positioned on an outer surface of the eye 10. The housing 102 is configured to receive the needle 20 therethrough to access the eye 10. The housing 102 can further be configured to connect to a negative pressure source 22. The negative pressure source is configured to provide a negative pressure to the ocular needle guide 100 via a pressure conduit 103. The negative pressure can provide a retention force between the housing 102 and the eye 10 to maintain the attachment of the ocular needle guide 100 on the eye 10.

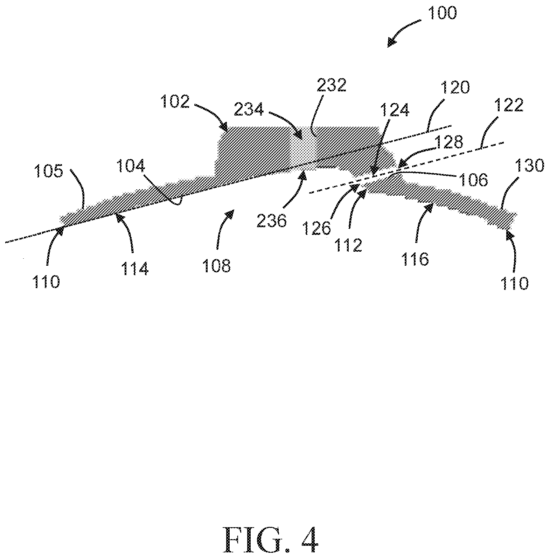

FIG. 4 illustrates a close-up view of the ocular needle guide 100 illustrated in FIG. 2. The housing 102 has a contact surface 104 configured to face and contact an exposed portion of the eye 10, and an outer surface 105 opposite the contact surface 104 in an outward direction, which also away from the eye 10. As will be described below, the contact surface is configured to contact the eye 10 so as to space an exposed portion of the sclera 14 from the choroid 12 to create a landing zone therebetween. The housing 102 further defines a needle guide channel 124 that extends from the outer surface 105 to the contact surface 104, and is configured to guide the needle into the eye into the landing zone along a direction that is substantially tangential to a surface of the exposed eye. The contact surface 104 has an outer peripheral edge 110 that extends about the housing 102. The outer peripheral edge 110 can be curved and extend circumferentially about the housing 102. In an aspect, the peripheral edge 110 extends continuously about the housing 102. The contact surface 104 defines a cavity 108 radially within the outer peripheral edge 110. The cavity 108 is sized to receive at least a portion of the eye 10 within.

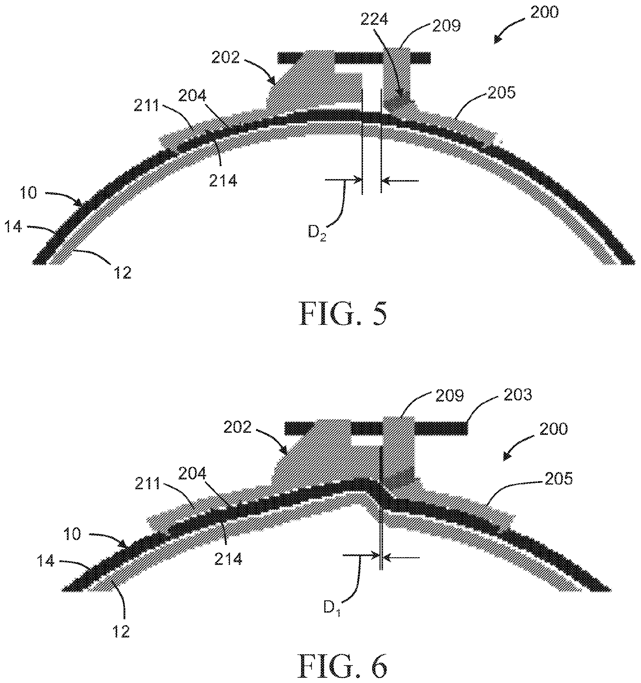

The contact surface 104 includes a first surface portion 112, a second surface portion 114, and a third surface portion 116. The third surface portion 116 can be referred to as a base surface portion that is configured to make contact with an undeformed portion of the sclera 14 that is undeformed, meaning that the third surface portion 116 does not space the sclera 14 from the choroid 12 when it contacts the sclera. The second surface portion 114 can define an ocular deformation surface portion that is configured to define a seat for a deformed portion of the sclera 14 that has been displaced under the suction force provided by the negative pressure source 22. In some examples, the sclera 14 can be displaced away from the choroid 12 to define a supra-choroidal landing zone. In other examples, the choroid 12 can be displaced along with the sclera 14 s as to define a sub-choroidal landing zone. In one example, a first or outer region of the second surface portion 114 can contact the undeformed sclera 14, and a second or inner region of the second surface portion 114 can define a seat for the deformed portion of the sclera 14 (see Fig. 5). The landing zone can be defined by the portion of the sclera 14 (alone or in combination with a portion of the choroid 12) that is deformed against the second region of the second surface portion 114. The first surface portion 112 can be referred to as a spacer surface portion that spaces the second surface portion 114 away from the third surface portion in the outward direction, and thus away from the eye 10. The first surface portion 112 can also define an inner opening to the needle guide channel 124.

The second surface portion 114 can extend at least partially along a contact plane 120. The contact plane 120 defines a plane that extends into and out of the page on FIG. 4. The second surface portion 114 can extend substantially along the contact plane 120 from the outer peripheral edge 110 radially inward toward the first surface portion 112. In an aspect, the entire second surface portion 114 extends substantially along the contact plane 120 from the outer peripheral edge 110 to the first surface portion 112. In some examples, the second surface portion 114 can be planar or curved as desired.

The ocular needle guide 100 can define a landing zone for the needle 20 when the contact surface 104 is placed against the eye 10. The term "landing zone" as used herein can refer to a zone that is disposed between the choroid 12 and deformed portion of the sclera 14 once the sclera 14 has been repositioned against the ocular needle guard 100, and is configured to receive an injection end of the needle 20. Thus, the landing zone can be partially defined by either or both of the outer peripheral edge 110 and the second surface portion 114. Specifically, either or both of the second surface portion 114 and the outer peripheral edge 110 at the second surface portion 114 can face the landing zone. More specifically, when the third surface portion 116 of the contact surface 104 is placed against the eye, a deformed portion of the sclera 14 is brought against the second surface portion 114, while the undeformed portion of the sclera 14 remains against the third surface portion 116. Thus, the landing zone can have a thickness that extends between a first distance and a second distance. The first distance can be defined from the second surface portion 114 to the choroidal facing surface of the deformed sclera 14. The second distance can be defined from the second surface portion 114 to the sclera 14. Thus, in one example the landing zone can be supra-choroidal. In other examples, the landing zone can be defined below the choroid 12, and can be referred to as sub-choroidal. The sub-choroidal landing zone can provide sub-choroidal access of a needle. The landing zone can have a length that extends along at least a portion of a circumferential length of the second surface portion 114 that extends from the first surface portion 112 to the outer peripheral edge 110 along the second surface portion 114. In one example, the length of the landing zone can extend between the second surface portion 114 and the outer peripheral edge 110. For instance, the length of the landing zone can extend from the second surface portion 114 toward the outer peripheral edge 110, and can terminate inward of the outer peripheral edge 110. In some examples, the length of the landing zone can extend from the second surface portion 114 to the outer peripheral edge 110.

The first surface portion 112 is angularly offset from the second surface portion 114 such that the cavity 108 is defined by the first surface portion 112 and the second surface portion 114. The first surface portion 112 can be curved or straight as desired. In an aspect, the first surface portion 112 can define an angle with a needle axis 122 as described below. The angle can be in a range from approximately 30 degrees to approximately 90 degrees, such as from approximately 45 degrees to approximately 90 degrees, such as from approximately 60 degrees to approximately 90 degrees, such as approximately 70 degrees to approximately 90 degrees, such as approximately 80 degrees to approximately 90 degrees. The angle can be defined by a straight line and the needle axis 122. In some examples whereby the first surface 112 is a flat surface, the straight line can extend along the first surface portion 112. In other examples, for instance when the first surface 112 is curved, the straight line can extend through a first point defined by an intersection of the first surface portion 112 and the third surface portion 116, and a second point defined by an intersection of the first surface portion 112 and the second surface portion 114.

The third surface portion 116 extends from the first surface portion 112 to the outer peripheral edge 110. The third surface portion 116 can be curved such that the third surface portion 116 approximates the curvature of the eye 10. The curvature of the third surface portion 116 can facilitate positioning the housing 102 on the eye 10. In one example, the housing 102 can be positioned on the eye 10 such that the eye extends along the third surface portion 116. The first surface portion 112 can define a standoff surface that extends outwardly from the third surface portion 116 to the second surface portion 114. As a result, the needle can enter the landing zone along a trajectory that is tangential to the eye or angularly offset from a direction that is tangential to the eye. At least an inner portion of the second surface portion 114 can therefore be spaced from the eye 10 when the third surface portion 116 contacts the eye. In this regard, the first surface portion 112 can be said to space the second surface portion 114 away from the third surface portion 116, and thus away from the eye during use, so as to define the landing zone described above. During use, the shape of the eye 10 can be remodeled by bringing a portion of the sclera 14 against the second surface portion 114 so as to define the landing zone described above. It should be appreciated that when the shape of the eye 10 is remodeled, the portion of the sclera 14 that is brought against the second surface portion 112 assumes the shape of the second surface portion 112. Another portion of the sclera 14 can extend along the third surface portion 116. Still another portion of the sclera 14 can extend along the first surface portion 112 so as to define a landing of the landing zone. The still another portion of the sclera 14 can define a landing of the landing zone that receive the needle 20 as the needle is driven to a location between the choroid 12 and the sclera 14. It will be appreciated that all embodiments of the ocular needle guide described herein can be configured to cause at least a portion of the sclera 14 to be repositioned by moving the portion of the sclera 14 away from the choroid 12 against a surface, thereby repositioning the sclera 14, and thus the eye 10. In particular, the portion of the sclera 14 can extend along at least a portion of the surface and thus can define the shape of the surface. The needle 20 can therefore be inserted between the portion of the choroid 12 and the sclera 14 in the landing zone.

It will be appreciated that the third surface portion 116 can be optional, such that the housing 102 includes the first surface portion 112 and the second surface portion 114, but not the third surface portion 116. For example, the first surface portion 112 can define the contact surface 104 that contacts the eye 10, and the second surface portion 114 extends from the first surface portion 112 a distance that is spaced outwardly away from the contact surface 104, and thus away from the eye during use. As described above, the shape of the eye 10 can be remodeled by bringing a portion of the sclera 14 against the second surface portion 112 so as to define the landing zone described above. It should be appreciated that when the shape of the eye 10 is remodeled, the portion of the sclera 14 that is brought against the second surface portion 112 assumes the shape of the second surface portion 112.

The housing 102 defines a needle guide surface 106 that extends about a needle axis 122 to define the needle guide channel 124. The needle guide channel 124 extends through the housing 102 to an opening 126 (e.g. a first opening) defined by the contact surface 104. The opening 126 of the needle guide channel 124 opens to the cavity 108 of the housing 102. In an aspect, the first surface portion 112 of the contact surface 104 defines the opening 126. The needle guide axis 122 can extend substantially parallel the contact plane 120 at a location adjacent to the contact plane, and can also extend along the contact plane 120. Thus, the needle guide axis 122 does not intersect the contact plane 120 within an outermost footprint of the ocular needle guide 100. In one example, the needle guide axis 122 can be parallel to the contact plane 120, or can be angularly offset from the contact plane 120 slightly but still substantially parallel to the contact plane 120 such that the needle 20 can be readily inserted through the sclera 14 and into the landing zone without penetrating the choroid 12. As used herein, the term substantially parallel can include an angle less than approximately twenty degrees, such as less than approximately ten degrees, such as less than approximately five degrees. The alignment of the needle guide axis 122 with the contact plane 120 facilitates the alignment of the needle 20 with the landing zone of the second surface portion 114 of the contact surface 104. The alignment of the needle 20 with the landing zone can provide a predictable depth of penetration of the needle 20 into the eye 10.

In an aspect, the needle axis 122 is substantially linear, such that the needle guide channel 124 extends linearly through the housing 102. Alternatively, the needle axis 122 can include a curved portion, such that a portion of the needle guide channel 124 is curved. For example, the needle guide channel 124 extends from an outer opening 228 defined by an outer surface 230 of the housing 102. The needle axis 122 can include a curved portion and a linear portion. The curved portion extending from the outer opening 128 to the linear portion, and the linear portion extending from the curved portion to the opening 126. The linear portion of the axis 122 can extend along the contact plane 120. The curved needle axis 122 can facilitate the alignment of a flexible needle (e.g. a nitinol needle) inserted through the needle guide channel 124 with the landing zone such that the flexible needle is substantially parallel to a direction of elongation of the landing zone.

The housing 102 can also include a pressure surface 232 that defines a pressure channel 234 that extends through the housing 102 from the outer surface 230 to a pressure opening 236 (e.g. a second opening) defined by the contact surface 104. The pressure opening 236 opens to the cavity 108 fluidly connecting an exterior of the housing 102 to the cavity 108. In an aspect, the pressure opening 236 is defined by the second surface portion 114 of the contact surface 104. The pressure channel 234 can be sized and configured to receive a pressure tube within. In an aspect, the pressure channel 234 can include a threaded region to threadedly connect to the pressure conduit 103. Alternatively, the pressure conduit 103 can be connected to the housing 102 via adhesives, glue, interference fit, snap-fit, or still other fasteners or connection means, to fluidly connect the negative pressure source 22 to the pressure channel 234.

Referring to FIGS. 2 and 4, the pressure conduit is connected to the housing 102 such that negative pressure source 22 is fluidly connected to the pressure channel 234. During an eye treatment procedure, the ocular needle guide 100 can be positioned on the eye 10 by a medical professional. The location of ocular needle guide 100 can be selected based on a desired treatment location on the eye 10. After placement of the ocular needle guide 100, a negative pressure force can be applied by the negative pressure source 22 to the cavity 108. The negative pressure force can draw the sclera 14 into the cavity 108. When the sclera 14 is drawn into the cavity 108, the outer surface of the eye 10 contacts the contact surface 104 of the housing 102 taking the approximate shape of the contact surface 104. The contact surface 104 defines a specific profile to deform the surface of the eye 10. The portion of the outer surface, or sclera 14, of the eye 10 that contacts the second surface portion 114, extends substantially parallel to the contact plane 120. The shaped surface of the second surface portion 114 can effectively flatten out at least a small portion of the sclera 14, such that the flattened portion of the sclera 14 is substantially parallel to the needle axis 122.

It will be appreciated that the ocular needle guide 100 can further include a handle (not shown). The handle can extend from the body 102 and manipulated by a user to adjust and locate the body 102 on the eye 10. The handle can be detachable from the body 102, such that when the body 102 is positioned on the eye 10, the handle can be removed prior to a therapeutic procedure. Alternatively, the handle can be fixed to the body 102 to help maintain the position of the body 102 on the eye 10 during the procedure.

With reference to FIGS. 3 and 4, once the eye 10 is contact with the contact surface 104 of the ocular needle guide 100 and the sclera is positioned within the cavity 108, the needle 20 can be inserted through the needle guide channel 124 in the housing 102 and directly into the landing zone. The configuration of the shaped surface of the second surface portion 114 and the needle guide channel 124 can make the depth of the needle penetration less critical than in tradition needle access. The needle 20 can include a needle stopper 24 to control the insertion depth such that the needle 20 does not extend beyond the landing zone and therefore does not penetrate the choroid 12.

The needle axis 122 can be offset from the contact plane 120 by a distance that substantially matches a thickness of the sclera 14 for supra-choroidal access, or a thickness of the sclera 14 and a thickness of a choroid 12 for sub-choroidal access. It will be appreciated that other distances for offsetting the needle 20 can be used to access other locations within the eye.

In an aspect, the needle 20 can benefit from a fast-firing mechanism, such as those used in biopsy needles. A quick action can mitigate the challenge of penetrating the tough sclera without losing the attachment of the ocular needle guide 100 to the eye 10. The biopsy type firing mechanism can also provide a predictable depth of penetration.

The needle 20 can have a needle tip with a conical or trocar type symmetrical shape that can facilitate the alignment of the needle 20 within they eye 10 as the needle 20 advances. The needle 20 can include side holes for injection. In an aspect, once the needle 20 has accessed the target location, a sheath or catheter (not shown) can be quickly left in place and the negative pressure can be removed from the cavity 108. This can provide access to the inter-layer space for further blunt dissection and/or delivery of a therapeutic agent.

A relatively quick procedure using the above describe method to deliver a therapeutic agent to an inner layer space of the eye 10 can involve placing the ocular needle guide 100 on the desired location of the eye 10, applying a vacuum from the negative pressure source 22, firing the needle 20, delivering the agent, removing the needle 20 from the eye 10, releasing the vacuum, and removing the ocular needle guide 100 from the eye 10. In an aspect, the "delivering the agent" step can be replaced with a catheter insertion step, to leave an access channel for additional deliveries to or navigations of the targeted space.

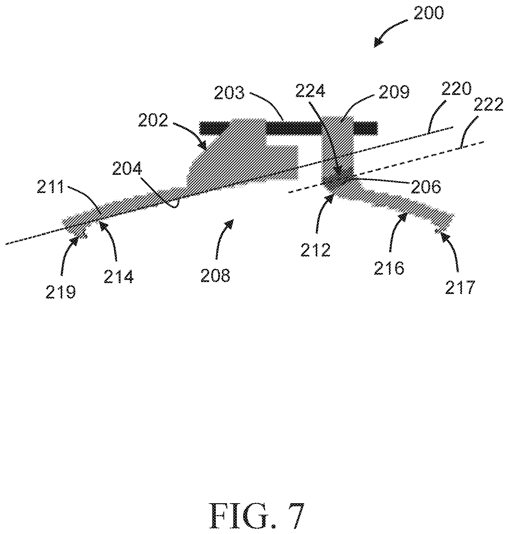

FIGS. 5 through 7 illustrate cross-sectional views of an alternate aspect of an ocular needle guide 200, according to aspects of this disclosure. Portions of the alternate aspect of the ocular needle guide 200 disclosed in FIGS. 5 through 7 are similar to aspects of the ocular needle guide 100 described above in FIGS. 1 through 4 and those portions function similarly to those described above. The ocular needle guide 200 includes a housing 202. The housing 202 is shaped to be positioned on the outer surface of the eye 10. The housing 202 is configured to receive the needle 20 therethrough to access the eye 10.

The housing 202 has a contact surface 204 and an outer surface 205 opposite the contact surface 204, and a needle guide channel 224 that extends from the outer surface 205 to the contact surface 204 as described above. The contact surface 204 defines a cavity 208 within the housing 202. The cavity 208 is sized to receive at least a portion of the eye 10 within.

The housing 202 includes a first housing portion 209 and a second housing portion 211. The contact surface 204 of the first housing portion 209 includes a first surface portion 212 and a third surface portion 216. The contact surface 204 of the second housing portion 211 includes a second surface portion 214. The second surface portion 214 extends at least partially along a contact plane 220. The contact surface 204 can define a landing zone of as described above. The third surface portion 216 can be referred to as a base surface portion that is configured to make contact with an undeformed portion of the sclera 14 that is undeformed, meaning that the third surface portion 216 does not space the sclera 14 from the choroid 12. The second surface portion 214 can be referred to as an ocular deformation surface portion that is configured to define a seat for a deformed portion of the sclera 14 that has been spaced away from the choroid 12 under the suction force provided by the negative pressure source 22. In this regard, the second surface portion 214 can be offset from the third surface portion 216 in the outward direction. The first surface portion 212 can extend from the third surface portion 216 in the outward direction so as to define an inner opening to the needle guide channel 224 that extends from the outer surface 205 to the first surface portion 212.

The housing 202 defines a needle guide surface 206 that extends about a needle axis 222 so as to define the needle guide channel 224. The needle guide channel 224 extends through the housing 202 to the contact surface 204 and opens to the cavity 208 of the housing 202. The needle guide axis 222 is substantially parallel to the contact plane 220. The alignment of the needle guide axis 222 with the contact plane 220 facilitates the alignment of the needle 20 with the landing zone.

The housing 202 can further include a first retention member 217 and a second retention member 219. The first retention member 217 can be positioned on the first housing portion 209. The second retention member 219 can be positioned on the second housing portion 211. The first and second retention members 217 and 219 can include, for example, opposing barbs, serrations, edges, protrusions, teeth, combinations thereof, or still other members extending from the contact surface 204 and capable of gripping the surface of the eye 10. It will be appreciated that the first and second retention members 217 and 219 can be formed as part of the respective first and second housing portions 209 and 211. Alternatively, the first and second retention members 217 and 219 can be separately attached to the contact surface 204 of the respective first and second housing portions 209 and 211. The first and second retention members 217 and 219 can each include a single respective retention member. Alternatively, the first and second retention members 217 and 219 can each include a plurality of retention members. The first and second retention members 217 and 219 can stabilize and hold the eye 10 to resist movement between the eye 10 and the ocular needle guide 100 when the ocular needle guide 100 is positioned on the eye 10.

With reference to FIGS. 5 and 6, the first housing portion 209 can be movable relative to the second housing portion 211 such that the housing 202 is transitionable between a retention position (FIG. 6) and an open position (FIG. 5). In the retention position, the first housing portion 209 is spaced from the second housing portion 211 by a first distance D₁. In the open position, the first housing portion 209 is spaced from the second housing portion 211 by a second distance D₂ that is greater than the first distance D₁. In an aspect, the needle guide channel 224 is spaced from the second surface portion 214 of the contact surface by a first distance in the retention position. In the open position, the needle guide channel 224 is spaced from the second surface portion 214 of the contact surface by a second distance that is greater than the first position. In an aspect, the first and second housing portions 209 and 211 can contact one another in the retention position, and the first and second housing portions 209 and 211 are spaced from each other in the open position.

In one example, the first housing portion 209 and the second housing portion 211 can be linearly movable relative to one another between the retention position and the open position. In particular, the ocular needle guide 200 can include a guide member 203 that is received by either or both of the first housing portion 209 and the second housing portion 211. Thus, either or both of the first and second housing portions 209 and 211 can selectively slide toward and away from the other of the first and second housing portions 209 and 211 along the guide member 203. The guide member 203 can extend along a central axis that can be straight or curved as desired. Either or both of the first and second housing portions 209 and 211 can travel along the central axis of the guide member 203 between the retention position and the open position. Thus, either or both of the first and second housing portions 209 and 211 can travel along the central axis of the guide member 203 along a straight path or a curved path between the retention position and the open position. The guide member 203 can be configured as a guide rod having any suitably shaped cross-section as desired.

When the ocular needle guide 200 is in the open position, the ocular needle guide 200 can be positioned on the eye 10 such that the contact surface 204 contacts the surface of the eye 10. After the ocular needle guide 200 is positioned on the eye 10, the housing 204 can be transitioned from the open position to the retention position, which can draw the sclera 14 into the cavity 208 of the housing 204 and against the second surface portion 214. For example, the first and second retention members 217 and 219 can engage the sclera 14 when the ocular needle guide 200 is positioned on the eye 10. As the distance between the first and second housing portion 209 and 211 transitions from the second distance D₂ to the first distance D₁, the first and second retention members 217 and 219 can pull the sclera 14 into the cavity 208, causing the surface, or sclera 14, of the eye 10 to contact the contact surface 204 and take the shape of the second surface portion 214. The portion of the outer surface of the eye 10 that contacts the second portion 214 can extend substantially parallel to the contact plane 220. The shaped surface of the second surface portion 214 can effectively flatten out at least a small portion of the sclera 14, such that the flattened portion of the sclera 14 is substantially parallel to the needle axis 222. This can remodel the tissue of the eye 10 by pulling the sclera into the cavity 208 to take the shape of the second surface portion 214 for shallow angle injections. The needle 20 can be inserted through the needle guide channel 224 in the housing 202 and directly into a target space below the sclera 14 in a substantially similar fashion as described above regarding the ocular needle guide 100.

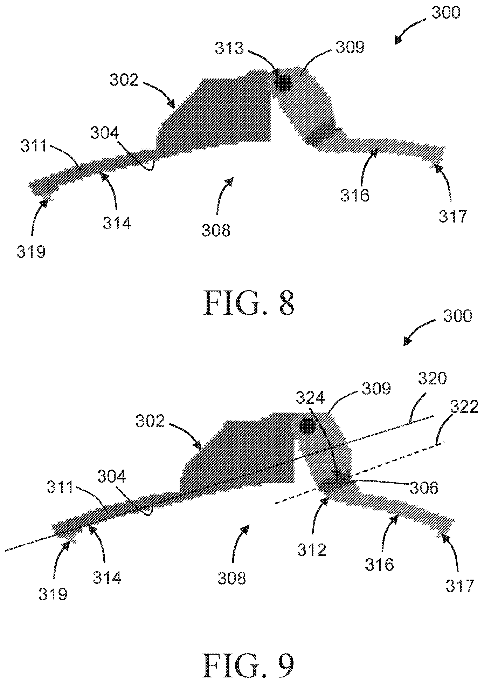

FIGS. 8 and 9 illustrate cross-sectional views of an alternate aspect of an ocular needle guide 300, according to aspects of this disclosure. Portions of the alternate aspect of the ocular needle guide 300 disclosed in FIGS. 8 and 9 are similar to aspects of the ocular needle guides 100 and 200 described above in FIGS. 1 through 7 and those portions function similarly to those described above. The ocular needle guide 300 includes a housing 302. The housing 302 is shaped to be positioned on the outer surface of the eye 10. The housing 302 is configured to receive the needle 20 therethrough to access the eye 10. The housing 302 has a contact surface 304 and an outer surface opposite the contact surface in the outward direction, and a needle guide surface 306 that defines a needle guide channel 324 in the manner described above. The contact surface 304 defines a cavity 308 within the housing 302. The cavity 308 is sized to receive at least a portion of the eye 10 within.

The housing 302 includes a first housing portion 309 and a second housing portion 311. The contact surface 304 of the first housing portion 309 includes a first surface portion 312 and a third surface portion 316. The contact surface 304 of the second housing portion 311 includes a second surface portion 314. The third surface portion 316 can be referred to as a base surface portion that is configured to make contact with an undeformed portion of the sclera 14 that is undeformed, meaning that the third surface portion 316 does not space the sclera 14 from the choroid 12. The second surface portion 314 can be referred to as an ocular deformation surface portion that is configured to define a seat for a deformed portion of the sclera 14 that has been spaced away from the choroid 12 under the suction force provided by the negative pressure source 22. In this regard, the second surface portion 314 can be offset from the first surface portion 312 in the outward direction. The first surface portion 312 can extend from the third surface portion 316 in the outward direction so as to define an inner opening to the needle guide channel 324 that extends from the outer surface 305 to the first surface portion 312. The second surface portion 314 extends at least partially along a contact plane 320. The contact surface 304, and in particular the second surface portion 314, can define a landing zone as described above.

The first housing portion 309 can be movable relative to the second housing portion 311 such that the housing 302 is transitionable between a retention position (FIG. 9) and an open position (FIG. 8). In an aspect, the first housing portion 309 is rotationally movable relative to the second housing portion 311 toward and away from each other, selectively, about a pivot member 313 between the retention position and the open position, respectively. The second and third surface portions 314 and 316 are spaced further from each other in the open position than the retention position. The pivot member 313 can include, for example, a pin, a rod, a ball, or still other member capable of facilitating rotation between components. In the open position, a needle guide axis 322 that defines a needle guide channel 324 is angularly offset from the contact plane 320. In the retention position, the needle guide axis 322 can be substantially parallel to the contact plane 320.

When the ocular needle guide 300 is in the open position, the ocular needle guide 300 can be positioned on the eye 10 such that the contact surface 304 contacts the surface of the eye 10. After the ocular needle guide 300 is positioned on the eye 10, the housing 304 can be transitioned from the open position to the retention position, which can draw the sclera 14 into the cavity 308 of the housing 304. For example, as the housing 304 is transitioned to the retention position, first and second retention members 317 and 319 can engage the sclera 14 and can pull the sclera 14 into the cavity 308. The force applied by the first and second retention members 317 and 319 can cause the sclera 14 of the eye 10 to contact the contact surface 304 so as to define the landing zone in the manner described above. The portion of the sclera 14 of the eye 10 that contacts the second surface portion 314, extends substantially parallel to the contact plane 320. The needle 20 can be inserted through the needle guide channel 324 in the housing 302 and directly into a target space below the sclera 14 in a substantially similar fashion as described above regarding the ocular needle guide 100.

It will be appreciated that the ocular needle guides 200 and 300 can also include other retention components or additional retention components. For example, the ocular needle guides 200 and 300 can include a vacuum (e.g. the negative pressure source 22) to maintain contact between the ocular needle guide 200 and 300 and the eye 10.

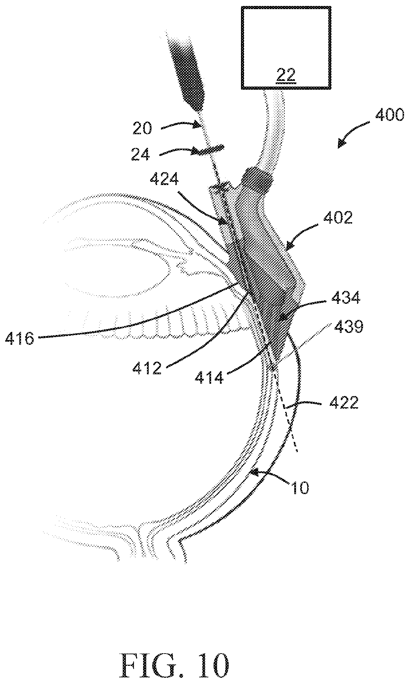

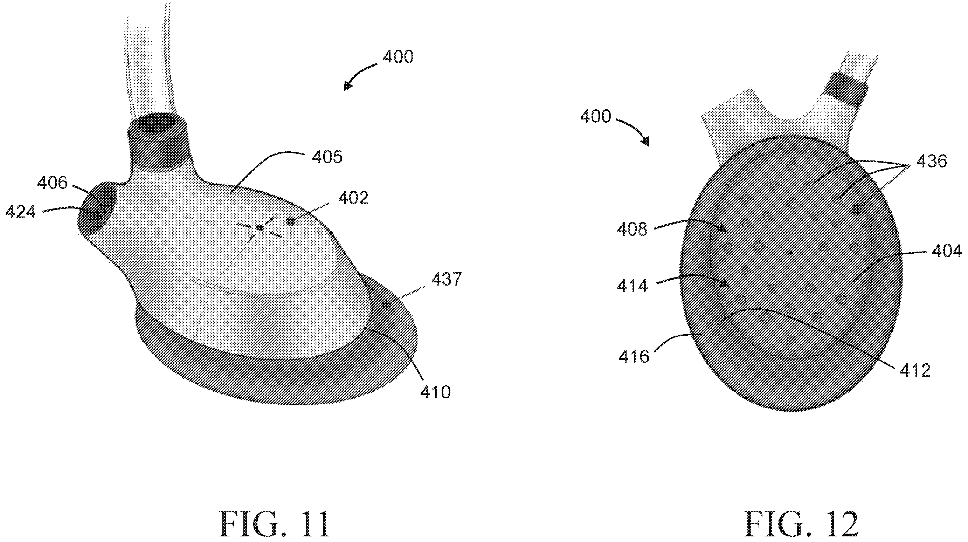

FIGS. 10 through 12 illustrate a cross-sectional view, a perspective view, and a bottom plan view, respectively, of an alternate aspect of an ocular needle guide 400, according to aspects of this disclosure. Portions of the alternate aspect of the ocular needle guide 400 disclosed in FIGS. 10 through 12 are similar to aspects of the ocular needle guides 100, 200, and 300 described above in FIGS. 1 through 9 and those portions function similarly to those described above. The ocular needle guide 400 includes a housing 402. The housing 402 is shaped to be positioned on the outer surface of the eye 10. The housing 402 is configured to receive the needle 20 therethrough to access the eye 10.

The housing 402 has a contact surface 404 and an outer surface 405 opposite the contact surface in the outward direction. The housing 402 further defines a needle guide channel 424 that extends from the outer surface 405 to the contact surface 405 in the manner described above. The contact surface 404 can further include a first surface portion 412, a second surface portion 414, and a third surface portion 416. The third surface portion 416 can be referred to as a base surface portion that is configured to make contact with an undeformed portion of the sclera 14 that is undeformed, meaning that the third surface portion 416 does not space the sclera 14 from the choroid 12. The second surface portion 414 can be referred to as an ocular deformation surface portion that is configured to define a seat for a deformed portion of the sclera 14 that has been spaced away from the choroid 12 under the suction force provided by the negative pressure source 22. In this regard, the second surface portion 414 can be offset from the first surface portion 412 in the outward direction. The first surface portion 412 can extend from the third surface portion 416 in the outward direction to the second surface portion 14. The needle guard channel 424 can extend from the outer surface 405 to the first surface portion 412, such that an inner opening to the needle guide channel 424 is disposed at the first surface portion 412. The contact surface 404 defines a cavity 408 within the housing 402. The cavity 408 is sized to receive at least a portion of the eye 10 within. The housing 402 defines needle guide surface 406 that, in turn, defines the needle guide channel 424 that is open to the cavity 408. The needle guide channel 424 extends about a needle guide axis 422. The needle guide axis 422 is substantially parallel to a direction of elongation of a landing zone that can be defined by the contact surface 404 in the manner described above.

The housing 402 can also include a plurality of pressure channels 434 that extend through the housing 402 from the outer surface 405 to the contact surface 404, and in particular to the second surface portion 414. The plurality of pressure channels 434 defines a corresponding plurality of pressure openings 436 at the second surface portion 414. Thus, the pressure channels 434 are open to the cavity 408 of the housing 402. The pressure openings 436 can be spaced about the contact surface 404. In aspect, each of the plurality of pressure openings 436 can be spaced approximately equidistant from each of the other pressure openings 436, or can be variably spaced as desired.

The ocular needle guide 400 can further include a gasket 437 that extends about an outer peripheral edge 410 of the housing 402. The gasket 437 is positioned to contact the eye 10 when the ocular needle guide is attached to the eye 10. The gasket 437 can assist in creating a seal between the housing 402 and the eye 10 when the suction force is applied to the cavity 408. In this regard, the gasket 437 can define both the third surface portion 416. The gasket 437 can further define the first surface portion 412 that extends from the third surface portion 416 to the second surface portion 414.

When the ocular needle guide 400 is positioned on the eye 10, the undeformed portion of the sclera 14 can contact the third contact surface 416. The negative pressure source 22 can define the suction force in the cavity 408. In particular, the negative pressure source 22 can induce a negative pressure in the cavity 408 that, in turn, creates the suction force that pulls the portion of the sclera 14 into the cavity 408 away from the choroid 12 and into contact with the contact surface 404, and in particular with the second surface portion 414. The portion of the sclera 14 that contacts the second surface portion 414 can take the shape of the second surface portion 414, and thus defines the deformed portion of the sclera 14. The deformed portion of the sclera 14 can thus be in a plane that is substantially parallel to the needle guide axis 422. The needle 20 can be inserted through the needle guide channel 424 in the housing 402, through the sclera 14, and into a target space 439 that defines the landing zone in an orientation that is substantially parallel to the second surface portion 414. The needle guide channel 424 can guide the entry of the needle into the suprachoroidal space. The needle 20 can include the needle stopper 24 to control the insertion depth such that the needle 20 does not extend beyond the landing zone and/or pass through the target space 439 and back into the sclera 14.

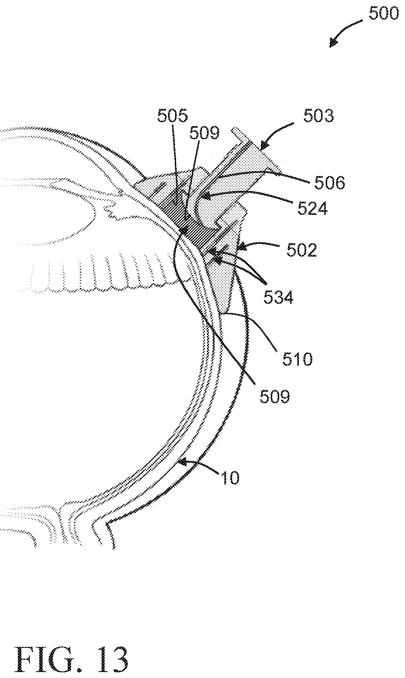

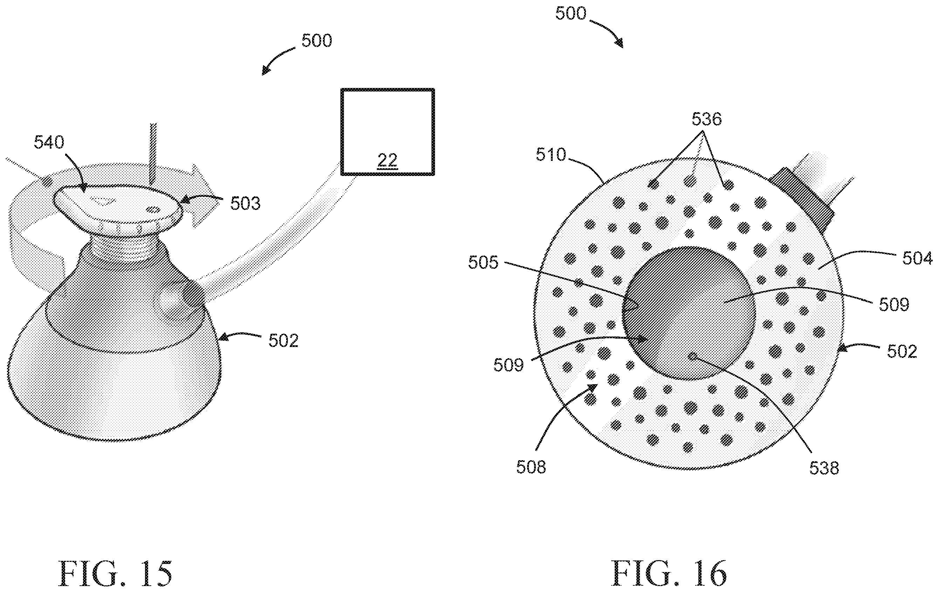

FIGS. 13 through 16 illustrate cross-sectional views of an alternate aspect of an ocular needle guide 500, according to aspects of this disclosure. Portions of the alternate aspect of the ocular needle guide 500 disclosed in FIGS. 13 through 16 are similar to aspects of the ocular needle guides 100, 200, 300, and 400 described above in FIGS. 1 through 12 and those portions function similarly to those described above. The ocular needle guide 500 includes a housing 502 and a post 503 at least partially positioned within the housing 502. The housing 502 is shaped to be positioned on the outer surface of the eye 10. The post 503 is configured to receive the needle 20 therethrough to access the eye 10.

The housing 502 has a first contact surface 504 and a post guide surface 505. The first contact surface 504 defines a cavity 508 within the housing 502. The cavity 508 is sized to receive at least a portion of the eye 10 within. In an aspect, the first contact surface 504 extends circumferentially within the housing 502 to define a substantially conical shape that tapers radially outwardly from the post guide surface 505 to an outer peripheral edge 510 of the housing 502. The first contact surface 504 can extend continuously about the housing 502 defining a 360 degree cone shape. In an alternative aspect, the first contact surface 504 defines at least a partially conical shape. The partially conical shape defining a cone shape of less than 360 degrees. The shape of the first contact surface 504 facilitates positioning the ocular needle guide 500 on the eye 10 and alignment of the needle 20 with the first contact surface 504.

The post guide surface 505 defines a post channel 508 within the housing 502. The post channel 509 is sized to receive at least a portion of the post 503 within. The post channel 509 extends through the housing 502 to an opening defined by the first contact surface 504 such that the post channel 509 opens to the cavity 508.

The housing 502 can also include a plurality of pressure channels 534 that extend through the housing 502 to a plurality of pressure openings 536. The plurality of pressure openings 536 are defined by the first contact surface 504.

The post 503 is positioned at least partially within the post channel 507. The post 503 has a second contact surface 509 and a needle guide surface 506. The needle guide surface 506 extends about a needle axis 522 through the post 503 to define a needle guide channel 524. The needle guide channel 524 has an opening 538 defined by the second contact surface 509. The needle axis 522 has a distal axis portion 523 that extends substantially linearly from a location within the need guide channel 524 through the opening 538 defined by the second contact surface 509. When the post 503 is positioned within the post channel 507, the distal axis portion 523 of the needle axis 522 is substantially parallel to at least a portion of the first contact surface 504 of the housing 502.

The post 503 is movable within the post channel 509. In an aspect, the post 503 is threadedly coupled within the post channel 509 such that rotation of the post 503 relative to the housing causes the post 503 to translate linearly through the post channel 509. The post 503 can be retracted to a position within the post channel 509 such that the second contact surface 509 is positioned within the post channel 509. The post 503 can be translated to a position within the post channel 509 such that the post 503 extends through the opening of the post channel 509 in first contact surface 504 and the second contact surface 509 is positioned within the cavity 508. The post 503 can be translated to different positions within the post channel 509 to define different depth positions to define needle depth positions. For example, the post 503 can translate to a first depth position within the cavity 508 and a second depth position within the cavity 508. In the first depth position, the distal axis portion 523 of the needle axis 522 is spaced from the substantially parallel portion of the first contact surface 504 by a first depth distance. The first depth distance can define a target depth within the eye 10. For example, the first depth distance can define an approximate thickness of a sclera 14 of a particular eye 10 such that a needle inserted through the needle guide channel 524 can be inserted into the eye 10 to a position between the sclera 14 and the choroid 12. The post 503 can be adjusted to different depth distances to accommodate various eye 10 sizes and configurations, and to modify the depth of injections. For example, the post 503 can be translated to a second depth position within the cavity 508 that is different than the first depth distance. The second depth position can define another target depth within the eye 10, for example, a sub-choroid location, or the second depth position can define a target depth for an eye having a different size and/or configuration than the eye 10.

When the post 503 is positioned within the cavity 508, the distal axis portion 523 of the needle axis 522 is spaced from the substantially parallel portion of the first contact surface 504 by a depth distance. As described above, the depth distance can be adjusted by moving the position of the second contact surface 509 of the post 503 within the cavity 508.

The post 503 can also include a direction indicator 540 positioned on a proximal end of the post. The direction indicator 540 can provide an indication to a user as to the direction in which the needle guide channel 524 opens into the cavity 508. The direction indicator 540 can include, for example, a specific shape of an end of the post (e.g. cam shape), a direction indication (e.g. an arrow), combinations thereof, or still other indicators to indicate a direction in which the needle guide channel 524 opens.

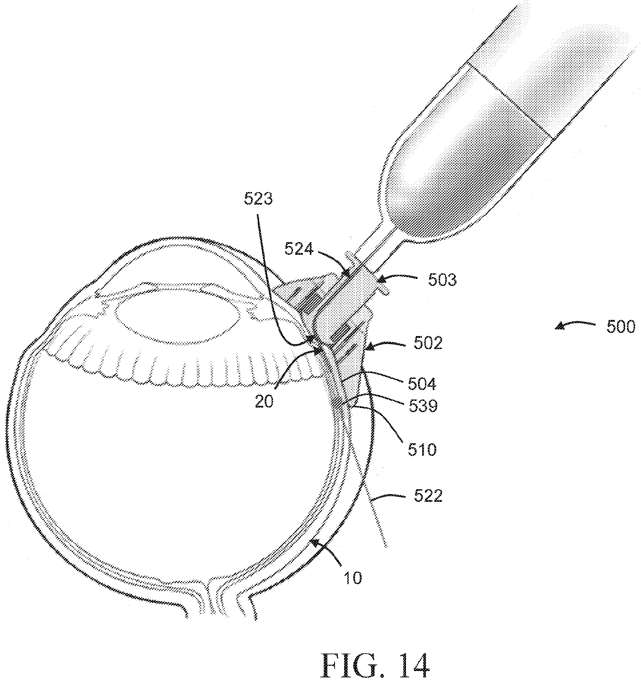

When the ocular needle guide 500 is positioned on the eye 10, a suction force can be applied by the negative pressure source 22 to the pressure channels 534 to pull the sclera 12 into the cavity 508 and into contact with the first contact surface 504 of the housing 502. The sclera 14 can take the shape of the first contact surface 504. The post 503 can be moved from a retracted position (FIG. 13) to an expanded position (FIG. 14) such that the second contact surface 509 of the post 503 is positioned within the cavity 508. After the post 503 is inserted into the cavity 508 to a desired depth position, the needle 20 can be inserted through the needle guide channel 524 in the post 503 and directly into a target space 539 within the eye 10 in an orientation that is substantially parallel to the first contact surface 504. The needle 20 can include the needle stopper 24 to control the insertion depth such that the needle 20 does not extend beyond the landing zone and/or pass through the target space 439 and back into the sclera 14.

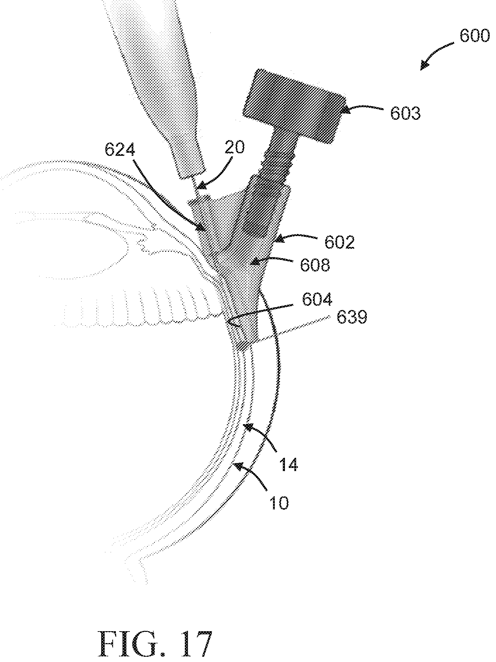

FIG. 17 illustrates a cross-sectional view of an alternate aspect of an ocular needle guide 600, according to an aspect of this disclosure. Portions of the alternate aspect of the ocular needle guide 600 disclosed in FIG. 17 are similar to aspects of the ocular needle guides 100, 200, 300, 400, and 500 described above in FIGS. 1 through 16 and those portions function similarly to those described above. The ocular needle guide 600 includes a housing 602 and a pressure post 603. The housing 602 is shaped to be positioned on the outer surface of the eye 10. The housing 602 is configured to receive the needle 20 therethrough to access the eye 10.

The housing 602 has a contact surface 604 and a needle guide channel 624. The needle guide channel 624 can be substantially parallel to the contact surface 604. The pressure post 603 is positionable within a pressure channel 608 defined by the housing 602. The pressure channel 608 opens to the contact surface 604. The pressure post 603 is translatable within the pressure channel 608 to affect a pressure force applied to the eye 10 when the eye 10 engages the contact surface 604. For example, when the contact surface 604 engages the eye 10, and the pressure post 603 is moved in a proximal direction away from the eye 10, a negative pressure (e.g. a vacuum) is created within the pressure channel 608 against the surface of the eye 10. The negative pressure can maintain the engagement between the eye 10 and the housing 602 during a therapeutic procedure. The negative pressure also helps achieve a deformation of the eye 10, creating a target space 639 (e.g. suprachoroidal space) within the eye 10 to release a therapeutic agent. To release the housing 602 from the eye 10, the pressure post 603 can be translated in a distal direction toward the eye 10 to reduce the vacuum force within the pressure channel 608. After the vacuum force is reduced, the housing 602 can be removed from the eye 10.

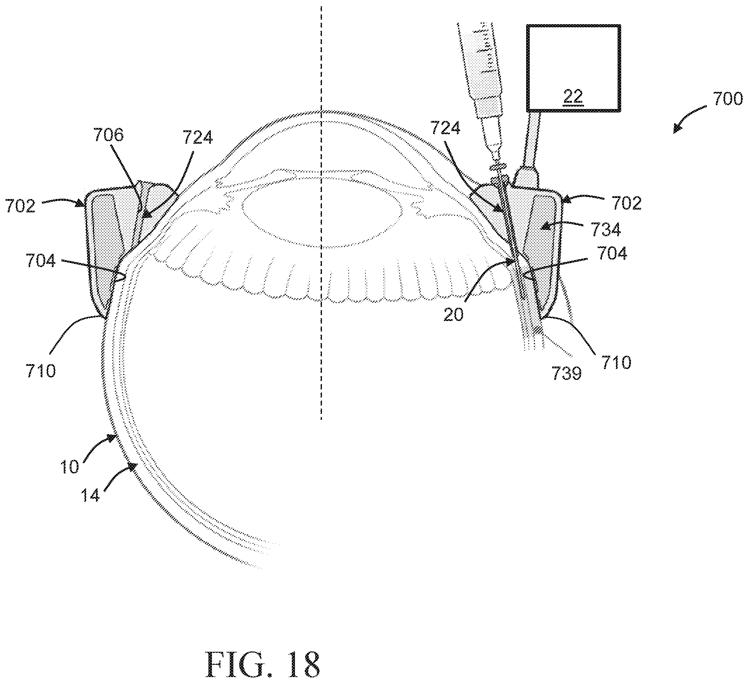

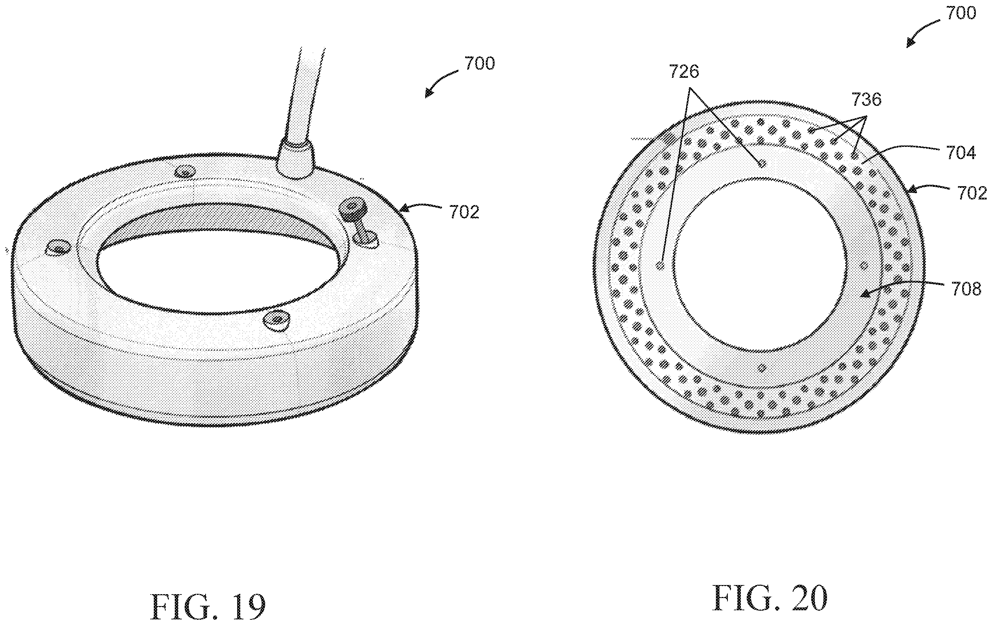

FIGS. 18 through 20 illustrate cross-sectional views of an alternate aspect of an ocular needle guide 700, according to aspects of this disclosure. Portions of the alternate aspect of the ocular needle guide 700 disclosed in FIGS. 18 through 20 are similar to aspects of the ocular needle guides 100, 200, 300, 400, 500, and 600 described above in FIGS. 1 through 17 and those portions function similarly to those described above. The ocular needle guide 700 includes a housing 702. The housing 702 is shaped to be positioned on the outer surface of the eye 10. The housing 702 is configured to receive the needle 20 therethrough to access the eye 10.

The housing 702 has a contact surface 704 and a plurality of needle guide surfaces 706. The contact surface 704 defines a cavity 708 within the housing 702. The cavity 708 is sized to receive at least a portion of the eye 10 within. The plurality of needle guide surfaces 706 define a plurality of needle guide channels 724 that open through a plurality of openings 726 into the cavity 708. Each needle guide channel 724 extend about a needle guide axis that is substantially parallel to a direction of elongation of a landing zone. For example, the contact surface 704 can extend circumferentially about a housing axis 745 of the housing 702. The housing axis 745 can extend through a center of the cavity 708. The contact surface 704 can taper outward along an anterior-posterior direction. Further, the contact surface 704 can define a substantially frustoconical shape that tapers radially outwardly from a position adjacent the needle guide surface 706 to an outer peripheral edge 710 of the housing 702. In other examples, the contact surface 704 can define any suitable shape other than frustoconical that is configured to remodel the eye 10 by repositioning the sclera 14, in particular at a location proximate to the peripheral edge 710 so as to define a region through which the needle is to be inserted into the landing zone. Each needle guide channel 724 can extend toward the cavity 708 in a direction that is substantially parallel to a portion of the contact surface 704 immediately adjacent to the respective opening 726 of the needle guide channel 724. Each needle guide channel 724 can extend in a direction that is offset from each of the other needle guide channels 724 while still being substantially parallel to the portion of the contact surface 704 immediately adjacent to the respective opening 726. In an aspect, each needle guide channel 724 can be spaced equidistant circumferentially about the housing 704. It will be appreciated that the housing 704 can include a single needle guide channel 724. In an aspect, the housing 702 comprises a lasik ring.

The housing 702 can also include at least one pressure channel 734 that extends through the housing 702 to a plurality of pressure openings 736. The plurality of pressure openings 736 are defined by the first contact surface 704.

When the ocular needle guide 700 is positioned on the eye 10, a suction force can be applied by the negative pressure source 22 to pull the sclera 12 into the cavity 708 and into contact with the contact surface 704. The sclera 14 can take the shape of the contact surface 704 such that the sclera 14 is substantially parallel to the direction the needle guide channel 724 extends toward the cavity 708. The needle 20 can be inserted through one of the needle guide channels 724 in the housing 702 and directly into a target space 739 below the sclera 14 in an orientation that is substantially parallel to the contact surface 704. The needle guide channel 724 can guide the entry of the needle into the suprachoroidal space. The needle 20 can include the needle stopper 24 to control the insertion depth such that the needle 20 does not extend beyond the contact surface 704 and/or pass through the target space 739 and back into the sclera 14.

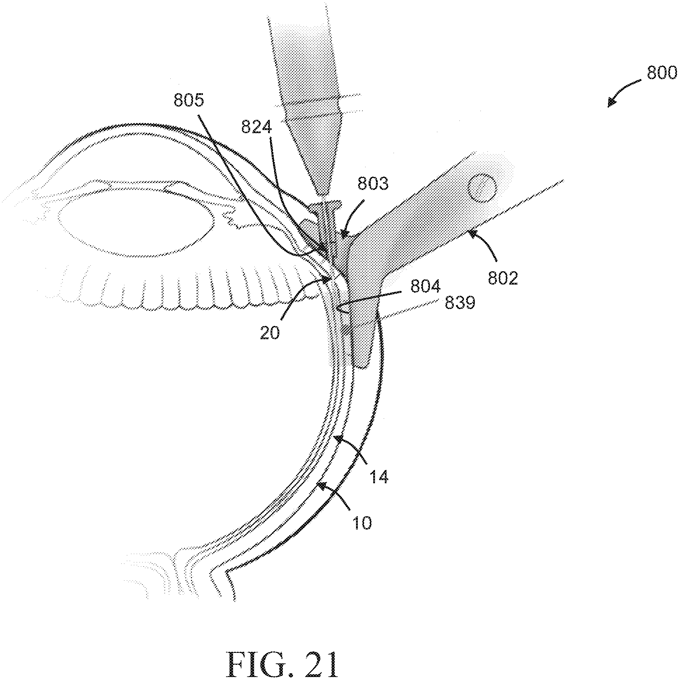

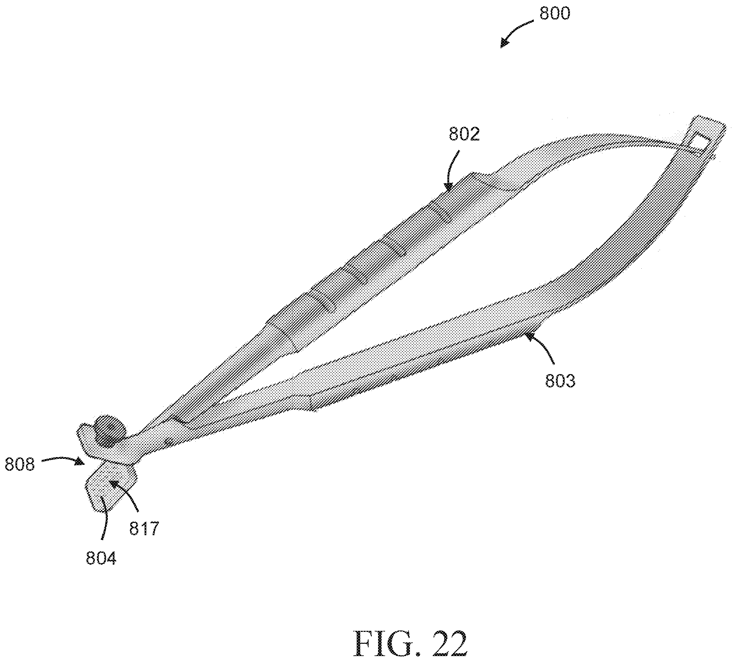

FIGS. 21 and 22 illustrate cross-sectional views of an alternate aspect of an ocular needle guide 800, according to aspects of this disclosure. Portions of the alternate aspect of the ocular needle guide 800 disclosed in FIGS. 21 and 22 are similar to aspects of the ocular needle guides 100, 200, 300, 400, 500, 600, and 700 described above in FIGS. 1 through 19 and those portions function similarly to those described above. The ocular needle guide 800 includes a first handle 802 and a second handle 803. The first and second handles 802 and 803 can be rotatably coupled together to define, for example, a pair of forceps. The first and second handles 802 and 803 being sized for grasping and manipulation by a user during a therapeutic procedure.

Each of the first and second handles 802 and 803 includes a respective first and second contact surface 804 and 805. The first and second contact surfaces 804 and 805 are shaped to be positioned on the outer surface of the eye 10. The first and second contact surfaces 804 and 805 can each include at least one retention member 817. The retention members 817 are configured to maintain engagement between the ocular needle guide 800 and the eye 10 during the therapeutic procedure. In aspect, each of the first and second contact surfaces 804 and 805 include a plurality of rejection members 817.

The second handle 803 defines a needle guide channel that extends through the second handle 803 to an opening defined by the second contact surface 805. The needle guide channel 824 is sized to receive the needle 20 therethrough and to access the eye 10.

The ocular needle guide 800 is transitionable between a retention position and an open position. In the retention position, the needle guide channel 824 is substantially parallel to at least a portion of the first contact surface 804. The first and second contact surfaces 804 and 805 define a cavity 808 sized to receive a portion of the sclera 14 within.

When the ocular needle guide 800 is in the open position, the ocular needle guide 800 can be positioned on the eye 10 such that the contact surfaces 804 and 805 contact the surface of the eye 10. After the ocular needle guide 800 is positioned on the eye 10, the ocular needle guide 800 can be transitioned from the open position to the retention position, which can cause the retention members 817 to draw the sclera 14 into the cavity 808. The force applied by the retention members 817 can cause the surface of the eye 10 to take the shape of the first and second contact surfaces 804 and 805. The portion of the outer surface of the eye 10 that contacts the first contact surface 804 extends substantially parallel to the needle guide channel 824. The needle 20 can be inserted through the needle guide channel 824 and directly into a target space 839 below the sclera 14 in a substantially similar fashion as described above regarding the ocular needle guide 100.

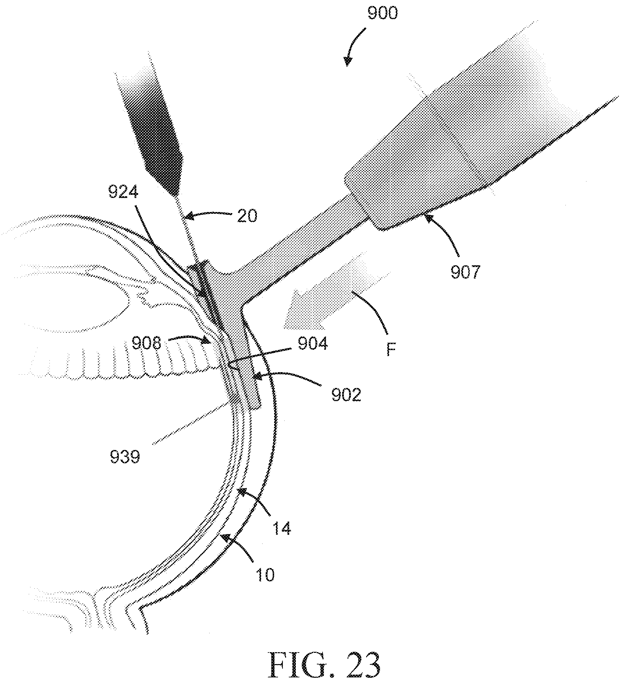

FIG. 23 illustrates a cross-sectional view of an alternate aspect of an ocular needle guide 900, according to aspects of this disclosure. Portions of the alternate aspect of the ocular needle guide 900 disclosed in FIG. 23 are similar to aspects of the ocular needle guides 100, 200, 300, 400, 500, 600, and 700 described above in FIGS. 1 through 21 and those portions function similarly to those described above. The ocular needle guide 900 includes a housing 902. The housing 902 is shaped to be positioned on the outer surface of the eye 10. The housing 902 is configured to receive the needle 20 therethrough to access the eye 10. The ocular needle guide 900 can include a handle 907 that can extend from the body 902. The handle 907 and manipulated by a user to adjust and locate the body 102 on the eye 10.

The housing 902 has a contact surface 904 and a needle guide channel 924 that opens into a cavity 908 defined by the contact surface 904. The needle guide channel 924 is substantially parallel to at least a portion of the contact surface 904.

When the ocular needle guide 900 is positioned on the eye 10, a force F can be applied by the user in a direction toward the eye 10. The applied force F can force the sclera 12 into the cavity 908 and to take the shape of the contact surface 904. The shape of the sclera 14 in contact with the contact surface 904 is substantially parallel to the needle guide channel 924. The needle 20 can be inserted through the needle guide channel 924 and directly into a target space 939 below the sclera 14 in an orientation that is substantially parallel to the contact surface 904. The needle guide channel 924 can guide the entry of the needle 20 into the suprachoroidal space.

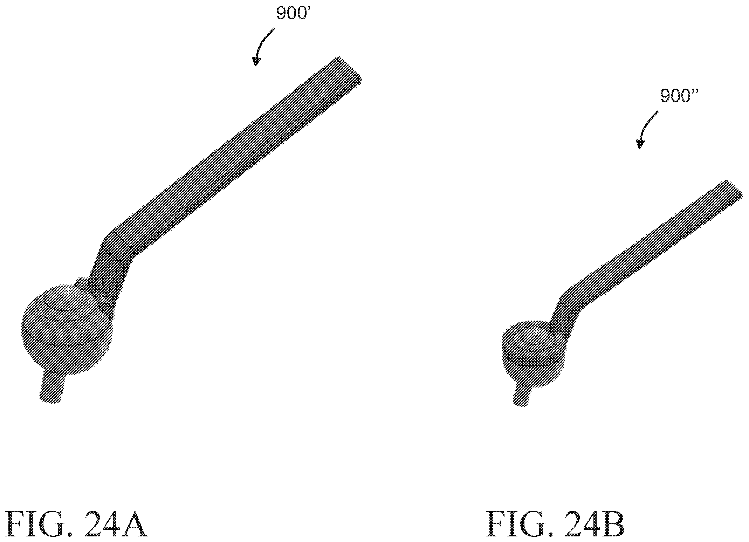

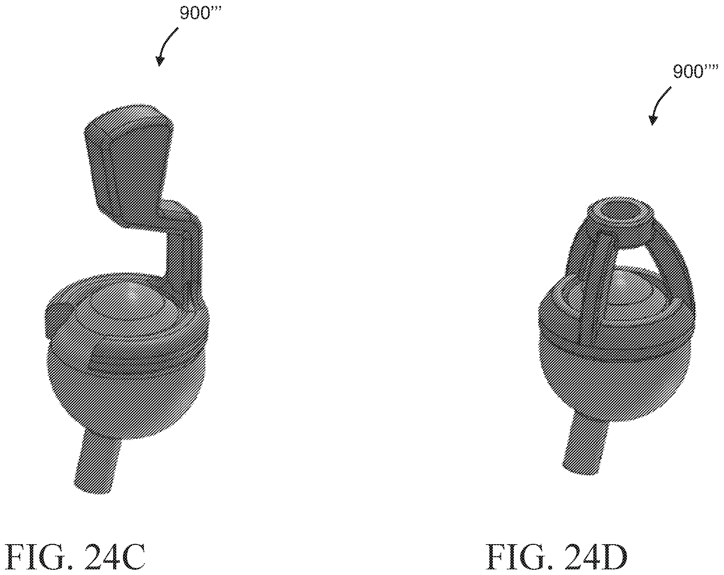

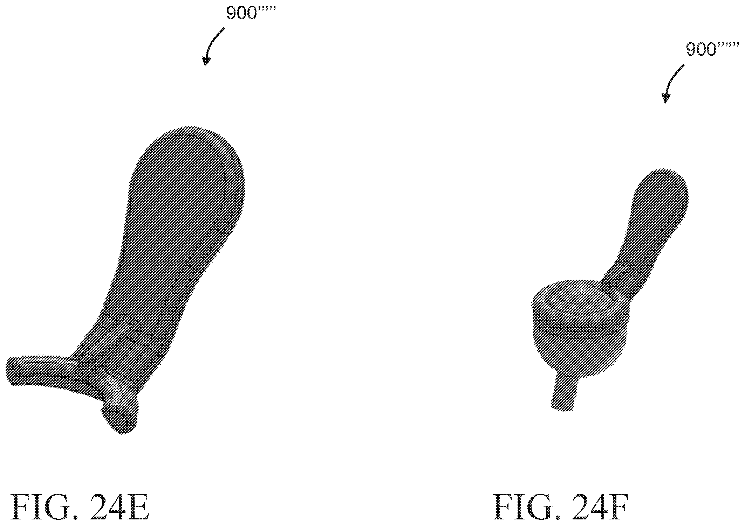

The ocular needle guide 900 can be referred to as a "press-fit" ocular needle guide. FIGS. 24A through 24F illustrate alternate aspects of press-fit ocular needle guides 900', 900", 900‴, 900ʺʺ, 900‴ʺ, and 900‴‴. Portions of the alternate aspects of the ocular needle guides disclosed in FIGS. 24A through 24F are similar to aspects of the ocular needle guide 900 described above in FIG. 23 and those portions function similarly to those described above. It will be appreciated that other configurations of press-fit ocular needle guides can include a needle guide channel and a contact surface that at least partially defines a landing zone of the type described above that is configured to receive a needle that injects a therapeutic agent into the eye 10. For example, a speculum press can include a needle guide channel and can be shaped to define a landing zone having a direction of elongation that is substantially parallel to the needle guide channel.

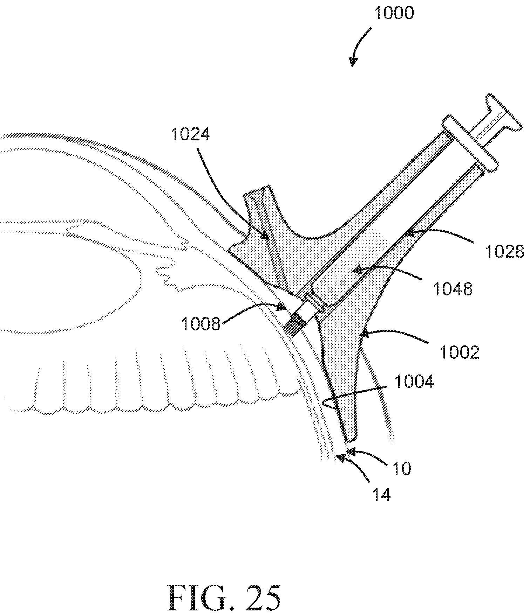

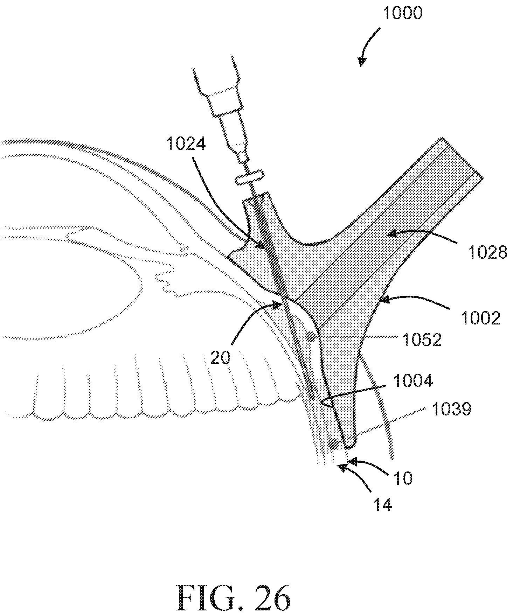

FIGS. 25 and 26 illustrate cross-sectional views of an alternate aspect of an ocular needle guide 1000, according to aspects of this disclosure. Portions of the alternate aspect of the ocular needle guide 1000 disclosed in FIGS. 25 and 26 are similar to aspects of the ocular needle guides 100, 200, 300, 400, 500, 600, 700, and 900 described above in FIGS. 1 through 20, 23, and 24A-24F and those portions function similarly to those described above. The ocular needle guide 1000 includes a housing 1002 that is shaped to be positioned on the outer surface of the eye 10.

The housing 1002 has a contact surface 1004, a needle guide channel 1024, and an injection fluid channel 1028. The needle guide channel 1024 is substantially parallel to a direction of elongation of a landing zone partially defined by the contact surface 1004. The contact surface 1004 defines a cavity 1008 within the housing 1002. The cavity 1008 is sized to receive at least a portion of the eye 10 within. The needle guide channel 1024 opens to the cavity 1008. The needle guide channel 1024 is substantially parallel to a direction of elongation of the landing zone 1005 that is partially defined by the contact surface 1004. The injection fluid channel 1028 extends through the housing 1002 and opens into the cavity 1008. The injection fluid channel 1028 has an opening defined by the contact surface 1004 radially between the needle guide channel 1024 and a portion of the contact surface 1004 that draws the sclera 14 away from the choroid 12. The injection fluid channel 1028 is sized to receive an agent injection device within to inject an agent into the eye 10.

When the ocular needle guide 1000 is positioned on the eye 10, a first injection device 1050 can be inserted into the injection fluid channel 1028. An agent 1048 can be injected into the eye 10 to create a bleb 1052 deforming the choroid 12 to cause the surface of the eye 10 push into the cavity 1008 of the body 1002. The sclera 14 can take the shape of the contact surface 1004, such that the sclera 14 is substantially parallel to the direction the needle guide channel 1024. The needle 20 can be inserted through the needle guide channel 1024 and through the bleb 1052 of agent 1048 within the eye 10 and directly into a target space 1039 below the sclera 14 in an orientation that is substantially parallel to a direction of elongation of the landing zone. The needle guide channel 1024 can guide the entry of the needle into the suprachoroidal space. The needle 20 can include the needle stopper 24 to control the insertion depth such that the needle 20 does not extend beyond the landing zone and/or pass through the target space 1039 and back into the sclera 14.

It will be appreciated that the foregoing description provides examples of the disclosed system and method. However, it is contemplated that other implementations of the disclosure may differ in detail from the foregoing examples. For example, any of the aspects disclosed herein can incorporate features disclosed with respect to any of the other aspects disclosed herein. All references to the disclosure or examples thereof are intended to reference the particular example being discussed at that point and are not intended to imply any limitation as to the scope of the disclosure more generally. All language of distinction and disparagement with respect to certain features is intended to indicate a lack of preference for those features, but not to exclude such from the scope of the disclosure entirely unless otherwise indicated.

As one of ordinary skill in the art will readily appreciate from that processes, machines, manufacture, composition of matter, means, methods, or steps, presently existing or later to be developed that perform substantially the same function or achieve substantially the same result as the corresponding embodiments described herein may be utilized according to the present disclosure.

## Claims

1. An ocular needle guide (200) configured to access a sub-choroidal space beneath a choroid (12) of an eye (10), or a supra-choroidal space between a sclera (14) of the eye and the choroid of the eye, the ocular needle guide comprising:
a housing (202) having a contact surface (204) and a needle guide surface (206),
the contact surface defining a cavity (208) within the housing, the contact surface extending at least partially along a contact plane (220),
the needle guide surface extending about a needle axis (222) to define a needle guide channel (224), the needle guide channel extending through the housing to an opening defined by the contact surface such that the needle guide channel opens to the cavity via the opening, the needle axis being substantially parallel to the contact plane,
wherein the ocular needle guide is configured to be positioned on the eye and pull a portion of the sclera away against a surface portion (214) of the contact surface.

2. The ocular needle guide of claim 1, wherein the housing further includes a pressure surface (232) that defines a pressure channel (234) extending through the housing to a second opening defined by the contact surface, such that the pressure channel opens to the cavity via the second opening.

3. The ocular needle guide of claim 2, wherein the pressure channel extends through the housing to a plurality of openings (436), the plurality of openings including the second opening and at least one other opening, wherein the pressure channel opens to the cavity via the plurality of openings.

4. The ocular needle guide of claim 2, wherein the contact surface has a first surface portion (212) and a second surface portion (214), the second surface portion extending along the contact plane, and the first surface portion being angularly offset from the contact plane such that the cavity is defined by the first surface portion and the second surface portion, wherein the first opening is defined by the first surface portion, and wherein the second opening is defined by the second surface portion; optionally wherein either:
an angle between the first surface portion and the second surface portion is less than 90 degrees; or
the contact surface further has a third surface portion (216) that extends from the first portion, the third surface portion being curved such that the third surface portion approximates the curvature of the eye.

5. The ocular needle guide of claim 1, wherein the contact surface extends between a first radially outermost end and a second radially outermost end, wherein the cavity is positioned between the first and second radially outmost ends, wherein the housing includes a first retention element (217) positioned adjacent to the first radially outermost end and a second retention element (219) positioned adjacent to the second radially outermost end, wherein when the surface of the eye contacts the contact surface, the first and second retention elements provide a retention force to retain the sclera within the cavity.

6. The ocular needle guide of claim 5, wherein the housing includes a first housing portion (209) and a second housing portion (211), wherein the first housing portion includes the first retention element, and wherein the second housing portion includes the second surface portion of the contact surface and the second retention element, the first housing portion being movable relative to the second housing portion such that the housing is transitionable between a retention position and an open position, wherein in the retention position the first retention member is spaced from the second retention member by a first distance, and wherein in the open position the first retention member is spaced from the second retention member by a second distance that is greater than the first distance;
optionally wherein the first housing portion is linearly movable relative to the second housing portion.

7. The ocular needle guide of claim 1, wherein the housing includes a first housing portion (209) and a second housing portion (211), the first housing portion including the needle guide channel, and the second housing portion including the portion of the contact surface extending along the contact plane,
wherein the needle axis extends through the opening of the contact surface and opposes the contact plane, and
wherein the first housing portion is movable relative to the second housing portion such that the housing is transitionable between a first depth position and a second depth position, wherein in the first depth position the needle axis is spaced away from the contact plane a first depth distance, and in the second depth position the needle axis is spaced away from the contact plane a second depth distance that is different than the first depth distance.

8. The ocular needle guide of claim 1, wherein the contact surface has an outer peripheral edge (410) that extends about the contact surface, wherein the ocular needle guide further comprises:
a gasket (437) coupled to the housing and extending radially outward from the outer peripheral edge of the contact surface, the gasket comprising a flexible material.

9. The ocular needle guide of claim 1, wherein the housing extends at least partially circumferentially about a housing axis, and wherein the contact surface extends at least partially in a conical shape.

10. The ocular needle guide of claim 9, wherein either:
the housing comprises a lasik ring; or
the needle guide surface is a first needle guide surface, the needle guide channel is a first needle guide channel, the needle axis is a first needle axis, the opening is a first needle opening, and the contact plane is a first contact plane,
wherein the contact surface extends at least partially along a second contact plane, and
wherein the housing further includes a second needle guide surface extending about a second needle guide axis to define a second needle guide channel, the second needle guide channel extending through the housing to a second needle opening defined by the contact surface such that the second needle guide channel opens to the cavity via the second needle opening, the needle axis being substantially parallel to the second contact plane.

11. The ocular needle guide of claim 1, configured to be positioned on the eye and pull a portion of the sclera away from the choroid into the cavity and against the surface portion of the contact surface.

12. A pharmaceutical kit comprising:
the ocular needle guide of claim 1; and
a needle (20) configured to be inserted through the needle guide channel along the needle axis.

13. The pharmaceutical kit of 12, wherein either:
a distal tip of the needle is cone shaped; or
a stopper (24) is coupled to the needle, the needle is insertable into the cavity of the housing to a predetermined distance, and the stopper is positioned to substantially prevent the needle from extending into the cavity a distance greater than the predetermined distance.

14. An ocular needle guide (300) for access into a space below a sclera (14) of an eye (10), the ocular needle guide comprising:
a first body portion (309) having a first contact surface (312, 316) and a needle guide surface (306), the needle guide surface extending about a needle axis (322) to define a needle guide channel (324), the needle guide channel extending through the housing to an opening defined by the first contact surface; and
a second body portion (311) having a second contact surface (314), the second contact surface extending at least partially along a contact plane (320),
the first body portion being movable relative to the second body portion such that the ocular needle guide is transitionable between an alignment position and an open position, wherein in the alignment position, the first contact surface and the second contact surface define a cavity, the needle guide channel opens to the cavity via the opening, and the needle axis is substantially parallel to the contact plane, and wherein in the open position, the needle axis is angularly offset from the contact plane.

15. An ocular needle guide (500) for access into a space below a sclera (14) of an eye (10), the ocular needle guide comprising:
a housing (502) having a first contact surface (504) and a post guide surface (505), the first contact surface defining a cavity (508) within the housing, and the post guide surface defining a post channel (509) within the housing; and
a post (503) positioned at least partially within the post channel, the post having a second contact surface (509) and a needle guide surface (506), the needle guide surface extending about a needle axis (522) through the post to define a needle guide channel (524), the needle guide channel having an opening (538) defined by the second contact surface, the needle axis having a distal axis portion (523) that extends substantially linearly from a location within the needle guide channel through the opening, the distal axis portion of the needle axis being substantially parallel to at least a portion of the first contact surface,
wherein the post is movable within the post channel to define a first depth position within the cavity and a second depth position within the cavity, wherein in the first depth position the distal axis portion of the needle axis is spaced from the substantially parallel portion of the first contact surface by a first depth distance, and in the second depth position the distal axis portion of the needle axis is spaced from the substantially parallel portion of the first contact surface by a second depth distance that is different than the first depth distance.

## Patentansprüche

1. Okulare Nadelführung (200), die konfiguriert ist, um auf einen subchoroidalen Raum unter einer Choroidea (12) eines Auges (10) oder einen suprachoroidalen Raum zwischen einer Sklera (14) des Auges und der Choroidea des Auges zuzugreifen, die okulare Nadelführung umfassend:
ein Gehäuse (202), das eine Kontaktoberfläche (204) und eine Nadelführungsoberfläche (206) aufweist,
wobei die Kontaktoberfläche einen Hohlraum (208) innerhalb des Gehäuses definiert, sich die Kontaktoberfläche mindestens teilweise entlang einer Kontaktebene (220) erstreckt,
die Nadelführungsoberfläche sich um eine Nadelachse (222) herum erstreckt, um einen Nadelführungskanal (224) zu definieren, wobei sich der Nadelführungskanal durch das Gehäuse hindurch zu einer Öffnung erstreckt, die durch die Kontaktoberfläche derart definiert ist, dass sich der Nadelführungskanal über die Öffnung zu dem Hohlraum öffnet, wobei die Nadelachse im Wesentlichen parallel zu der Kontaktebene ist,
wobei die okulare Nadelführung konfiguriert ist, um auf dem Auge positioniert zu werden und einen Abschnitt der Sklera gegen einen Oberflächenabschnitt (214) der Kontaktoberfläche wegzuziehen.

2. Okulare Nadelführung nach Anspruch 1, wobei das Gehäuse ferner eine Druckoberfläche (232) einschließt, die einen Druckkanal (234) definiert, der sich durch das Gehäuse hindurch zu einer zweiten Öffnung erstreckt, die durch die Kontaktoberfläche derart definiert ist, dass sich der Druckkanal über die zweite Öffnung zu dem Hohlraum öffnet.

3. Okulare Nadelführung nach Anspruch 2, wobei sich der Druckkanal durch das Gehäuse hindurch zu einer Vielzahl von Öffnungen (436) erstreckt, wobei die Vielzahl von Öffnungen die zweite Öffnung und mindestens eine weitere Öffnung einschließt, wobei sich der Druckkanal über die Vielzahl von Öffnungen zu dem Hohlraum öffnet.

4. Okulare Nadelführung nach Anspruch 2, wobei die Kontaktoberfläche einen ersten Oberflächenabschnitt (212) und einen zweiten Oberflächenabschnitt (214) aufweist, wobei sich der zweite Oberflächenabschnitt entlang der Kontaktebene erstreckt und der erste Oberflächenabschnitt von der Kontaktebene derart winkelversetzt ist, dass der Hohlraum durch den ersten Oberflächenabschnitt und den zweiten Oberflächenabschnitt definiert ist, wobei die erste Öffnung durch den ersten Oberflächenabschnitt definiert ist und wobei die zweite Öffnung durch den zweiten Oberflächenabschnitt definiert ist; wobei optional entweder:
ein Winkel zwischen dem ersten Oberflächenabschnitt und dem zweiten Oberflächenabschnitt kleiner als 90 Grad ist; oder
die Kontaktoberfläche ferner einen dritten Oberflächenabschnitt (216) aufweist, der sich von dem ersten Abschnitt erstreckt, wobei der dritte Oberflächenabschnitt derart gekrümmt ist, dass der dritte Oberflächenabschnitt sich der Krümmung des Auges annähert.

5. Okulare Nadelführung nach Anspruch 1, wobei sich die Kontaktoberfläche zwischen einem ersten radial äußersten Ende und einem zweiten radial äußersten Ende erstreckt, wobei der Hohlraum zwischen dem ersten und dem zweiten radial äußersten Ende positioniert ist, wobei das Gehäuse ein erstes Halteelement (217), das angrenzend an das erste radial äußerste Ende positioniert ist, und ein zweites Halteelement (219) einschließt, das angrenzend an das zweite radial äußerste Ende positioniert ist, wobei, wenn die Oberfläche des Auges die Kontaktoberfläche kontaktiert, das erste und das zweite Halteelement eine Haltekraft bereitstellen, um die Sklera innerhalb des Hohlraums zu halten.

6. Okulare Nadelführung nach Anspruch 5, wobei das Gehäuse einen ersten Gehäuseabschnitt (209) und einen zweiten Gehäuseabschnitt (211) einschließt, wobei der erste Gehäuseabschnitt das erste Halteelement einschließt, und wobei der zweite Gehäuseabschnitt den zweiten Oberflächenabschnitt der Kontaktoberfläche und das zweite Halteelement einschließt, wobei der erste Gehäuseabschnitt relativ zu dem zweiten Gehäuseabschnitt derart bewegbar ist, dass das Gehäuse zwischen einer Halteposition und einer offenen Position überführbar ist, wobei in der Halteposition das erste Halteglied von dem zweiten Halteglied um einen ersten Abstand beabstandet ist, und wobei in der offenen Position das erste Halteglied von dem zweiten Halteglied um einen zweiten Abstand beabstandet ist, der größer als der erste Abstand ist;
optional wobei der erste Gehäuseabschnitt relativ zu dem zweiten Gehäuseabschnitt linear bewegbar ist.

7. Okulare Nadelführung nach Anspruch 1, wobei das Gehäuse einen ersten Gehäuseabschnitt (209) und einen zweiten Gehäuseabschnitt (211) einschließt, wobei der erste Gehäuseabschnitt den Nadelführungskanal einschließt und der zweite Gehäuseabschnitt den Abschnitt der Kontaktoberfläche einschließt, der sich entlang der Kontaktebene erstreckt,
wobei sich die Nadelachse durch die Öffnung der Kontaktoberfläche hindurch erstreckt und der Kontaktebene gegenüberliegt, und
wobei der erste Gehäuseabschnitt relativ zu dem zweiten Gehäuseabschnitt derart bewegbar ist, dass das Gehäuse zwischen einer ersten Tiefenposition und einer zweiten Tiefenposition überführbar ist, wobei in der ersten Tiefenposition die Nadelachse von der Kontaktebene um einen ersten Tiefenabstand beabstandet ist und in der zweiten Tiefenposition die Nadelachse von der Kontaktebene um einen zweiten Tiefenabstand beabstandet ist, der sich von dem ersten Tiefenabstand unterscheidet.

8. Okulare Nadelführung nach Anspruch 1, wobei die Kontaktoberfläche einen äußeren Umfangsrand (410) aufweist, der sich um die Kontaktoberfläche erstreckt, wobei die okulare Nadelführung ferner umfasst:
eine Dichtung (437), die mit dem Gehäuse gekoppelt ist und sich von dem äußeren Umfangsrand der Kontaktoberfläche radial nach außen erstreckt, die Dichtung umfassend ein flexibles Material.

9. Okulare Nadelführung nach Anspruch 1, wobei sich das Gehäuse mindestens teilweise umlaufend um eine Gehäuseachse herum erstreckt, und wobei sich die Kontaktoberfläche mindestens teilweise in einer konischen Form erstreckt.

10. Okulare Nadelführung nach Anspruch 9, wobei entweder:
das Gehäuse einen Lasik-Ring umfasst; oder
die Nadelführungsoberfläche eine erste Nadelführungsoberfläche ist, der Nadelführungskanal ein erster Nadelführungskanal ist, die Nadelachse eine erste Nadelachse ist, die Öffnung eine erste Nadelöffnung ist und die Kontaktebene eine erste Kontaktebene ist,
wobei sich die Kontaktoberfläche mindestens teilweise entlang einer zweiten Kontaktebene erstreckt, und
wobei das Gehäuse ferner eine zweite Nadelführungsoberfläche einschließt, die sich um eine zweite Nadelführungsachse herum erstreckt, um einen zweiten Nadelführungskanal zu definieren, wobei sich der zweite Nadelführungskanal durch das Gehäuse hindurch zu einer zweiten Nadelöffnung erstreckt, die durch die Kontaktoberfläche derart definiert ist, dass sich der zweite Nadelführungskanal über die zweite Nadelöffnung zu dem Hohlraum öffnet, wobei die Nadelachse im Wesentlichen parallel zu der zweiten Kontaktebene ist.

11. Okulare Nadelführung nach Anspruch 1, die konfiguriert ist, um auf dem Auge positioniert zu werden und einen Abschnitt der Sklera von der Choroidea weg in den Hohlraum und gegen den Oberflächenabschnitt der Kontaktoberfläche zu ziehen.

12. Pharmazeutisches Kit, umfassend:
die okulare Nadelführung nach Anspruch 1; und
eine Nadel (20), die konfiguriert ist, um durch den Nadelführungskanal hindurch entlang der Nadelachse eingeführt zu werden.

13. Pharmazeutisches Kit nach 12, wobei entweder:
eine distale Spitze der Nadel kegelförmig ist; oder
ein Stopfen (24) mit der Nadel gekoppelt ist, die Nadel in den Hohlraum des Gehäuses bis zu einer vorbestimmten Distanz einführbar ist, und der Stopfen positioniert ist, um im Wesentlichen zu verhindern, dass sich die Nadel um eine Distanz in den Hohlraum erstreckt, die größer als die vorbestimmte Distanz ist.

14. Okulare Nadelführung (300) für einen Zugang in einen Raum unter einer Sklera (14) eines Auges (10), die okulare Nadelführung umfassend:
einen ersten Körperabschnitt (309), der eine erste Kontaktoberfläche (312, 316) und eine Nadelführungsoberfläche (306) aufweist, wobei sich die Nadelführungsoberfläche um eine Nadelachse (322) herum erstreckt, um einen Nadelführungskanal (324) zu definieren, wobei sich der Nadelführungskanal durch das Gehäuse hindurch zu einer Öffnung erstreckt, die durch die erste Kontaktoberfläche definiert ist; und
einen zweiten Körperabschnitt (311), der eine zweite Kontaktoberfläche (314) aufweist, wobei sich die zweite Kontaktoberfläche mindestens teilweise entlang einer Kontaktebene (320) erstreckt,
wobei der erste Körperabschnitt relativ zu dem zweiten Körperabschnitt derart bewegbar ist, dass die okulare Nadelführung zwischen einer Ausrichtungsposition und einer offenen Position überführbar ist, wobei in der Ausrichtungsposition die erste Kontaktoberfläche und die zweite Kontaktoberfläche einen Hohlraum definieren, sich der Nadelführungskanal über die Öffnung zu dem Hohlraum öffnet und die Nadelachse im Wesentlichen parallel zu der Kontaktebene ist, und wobei in der offenen Position die Nadelachse von der Kontaktebene winkelversetzt ist.

15. Okulare Nadelführung (500) für einen Zugang in einen Raum unter einer Sklera (14) eines Auges (10), die okulare Nadelführung umfassend:
ein Gehäuse (502), das eine erste Kontaktoberfläche (504) und eine Pfostenführungsoberfläche (505) aufweist, wobei die erste Kontaktoberfläche einen Hohlraum (508) innerhalb des Gehäuses definiert und die Pfostenführungsoberfläche einen Pfostenkanal (509) innerhalb des Gehäuses definiert; und
einen Pfosten (503), der mindestens teilweise innerhalb des Pfostenkanals positioniert ist, wobei der Pfosten eine zweite Kontaktoberfläche (509) und eine Nadelführungsoberfläche (506) aufweist, wobei sich die Nadelführungsoberfläche um eine Nadelachse (522) herum durch den Pfosten hindurch erstreckt, um einen Nadelführungskanal (524) zu definieren, wobei der Nadelführungskanal eine Öffnung (538) aufweist, die durch die zweite Kontaktoberfläche definiert ist, wobei die Nadelachse einen distalen Achsenabschnitt (523) aufweist, der sich im Wesentlichen linear von einer Stelle innerhalb des Nadelführungskanals durch die Öffnung hindurch erstreckt, wobei der distale Achsenabschnitt der Nadelachse im Wesentlichen parallel zu mindestens einem Abschnitt der ersten Kontaktoberfläche ist,
wobei der Pfosten innerhalb des Pfostenkanals bewegbar ist, um eine erste Tiefenposition innerhalb des Hohlraums und eine zweite Tiefenposition innerhalb des Hohlraums zu definieren, wobei in der ersten Tiefenposition der distale Achsenabschnitt der Nadelachse von dem im Wesentlichen parallelen Abschnitt der ersten Kontaktoberfläche um einen ersten Tiefenabstand beabstandet ist, und in der zweiten Tiefenposition der distale Achsenabschnitt der Nadelachse von dem im Wesentlichen parallelen Abschnitt der ersten Kontaktoberfläche um einen zweiten Tiefenabstand beabstandet ist, der sich von dem ersten Tiefenabstand unterscheidet.

## Revendications

1. Guide d'aiguille oculaire (200) conçu pour accéder à un espace sous-choroïdien sous une choroïde (12) d'un œil (10), ou à un espace supra-choroïdien entre une sclérotique (14) de l'œil et la choroïde de l'œil, le guide d'aiguille oculaire comprenant :
un boîtier (202) ayant une surface de contact (204) et une surface de guidage d'aiguille (206),
la surface de contact définissant une cavité (208) au sein du boîtier, la surface de contact s'étendant au moins partiellement le long d'un plan de contact (220),
la surface de guidage d'aiguille s'étendant autour d'un axe d'aiguille (222) pour définir un canal de guidage d'aiguille (224), le canal de guidage d'aiguille s'étendant à travers le boîtier jusqu'à une ouverture définie par la surface de contact de telle sorte que le canal de guidage d'aiguille débouche dans la cavité par le biais de l'ouverture, l'axe d'aiguille étant sensiblement parallèle au plan de contact,
dans lequel le guide d'aiguille oculaire est conçu pour être positionné sur l'œil et tirer une partie de la sclérotique à l'écart contre une partie de surface (214) de la surface de contact.

2. Guide d'aiguille oculaire selon la revendication 1, dans lequel le boîtier comporte en outre une surface de pression (232) qui définit un canal de pression (234) s'étendant à travers le boîtier jusqu'à une seconde ouverture définie par la surface de contact, de telle sorte que le canal de pression débouche dans la cavité par le biais de la seconde ouverture.

3. Guide d'aiguille oculaire selon la revendication 2, dans lequel le canal de pression s'étend à travers le boîtier jusqu'à une pluralité d'ouvertures (436), la pluralité d'ouvertures comportant la seconde ouverture et au moins une autre ouverture, dans lequel le canal de pression débouche dans la cavité par le biais de la pluralité d'ouvertures.

4. Guide d'aiguille oculaire selon la revendication 2, dans lequel la surface de contact a une première partie de surface (212) et une deuxième partie de surface (214), la deuxième partie de surface s'étendant le long du plan de contact, et la première partie de surface étant décalée angulairement par rapport au plan de contact de telle sorte que la cavité est définie par la première partie de surface et la deuxième partie de surface, dans lequel la première ouverture est définie par la première partie de surface, et dans lequel la seconde ouverture est définie par la deuxième partie de surface ; facultativement dans lequel soit :
un angle entre la première partie de surface et la deuxième partie de surface est inférieur à 90 degrés ; soit
la surface de contact a en outre une troisième partie de surface (216) qui s'étend à partir de la première partie, la troisième partie de surface étant incurvée de telle sorte que la troisième partie de surface se rapproche de la courbure de l'œil.

5. Guide d'aiguille oculaire selon la revendication 1, dans lequel la surface de contact s'étend entre une première extrémité radialement la plus externe et une seconde extrémité radialement la plus externe, dans lequel la cavité est positionnée entre les première et seconde extrémités radialement les plus externes, dans lequel le boîtier comporte un premier élément de retenue (217) positionné de manière adjacente à la première extrémité radialement la plus externe et un second élément de retenue (219) positionné de manière adjacente à la seconde extrémité radialement la plus externe, dans lequel, lorsque la surface de l'œil entre en contact avec la surface de contact, les premier et second éléments de retenue fournissent une force de retenue pour retenir la sclérotique au sein de la cavité.

6. Guide d'aiguille oculaire selon la revendication 5, dans lequel le boîtier comporte une première partie de boîtier (209) et une seconde partie de boîtier (211), dans lequel la première partie de boîtier comporte le premier élément de retenue, et dans lequel la seconde partie de boîtier comporte la seconde partie de surface de la surface de contact et le second élément de retenue, la première partie de boîtier étant mobile par rapport à la seconde partie de boîtier de telle sorte que le boîtier peut effectuer une transition entre une position de retenue et une position ouverte, dans lequel, dans la position de retenue, le premier élément de retenue est espacé du second élément de retenue d'une première distance, et dans lequel, dans la position ouverte, le premier élément de retenue est espacé du second élément de retenue d'une seconde distance qui est supérieure à la première distance ;
facultativement dans lequel la première partie de boîtier est mobile linéairement par rapport à la deuxième partie de boîtier.

7. Guide d'aiguille oculaire selon la revendication 1, dans lequel le boîtier comporte une première partie de boîtier (209) et une seconde partie de boîtier (211), la première partie de boîtier comportant le canal de guidage d'aiguille, et la seconde partie de boîtier comportant la partie de la surface de contact s'étendant le long du plan de contact,
dans lequel l'axe d'aiguille s'étend à travers l'ouverture de la surface de contact et s'oppose au plan de contact, et
dans lequel la première partie de boîtier est mobile par rapport à la seconde partie de boîtier de telle sorte que le boîtier peut effectuer une transition entre une première position de profondeur et une seconde position de profondeur, dans lequel, dans la première position de profondeur, l'axe d'aiguille est espacé du plan de contact d'une première distance de profondeur, et dans la seconde position de profondeur, l'axe d'aiguille est espacé du plan de contact d'une seconde distance de profondeur qui est différente de la première distance de profondeur.

8. Guide d'aiguille oculaire selon la revendication 1, dans lequel la surface de contact a un bord périphérique externe (410) qui s'étend autour de la surface de contact, dans lequel le guide d'aiguille oculaire comprend en outre :
un joint (437) accouplé au boîtier et s'étendant radialement vers l'extérieur à partir du bord périphérique externe de la surface de contact, le joint comprenant un matériau flexible.

9. Guide d'aiguille oculaire selon la revendication 1, dans lequel le boîtier s'étend au moins partiellement circonférentiellement autour d'un axe de boîtier, et dans lequel la surface de contact s'étend au moins partiellement selon une forme conique.

10. Guide d'aiguille oculaire selon la revendication 9, dans lequel soit :
le boîtier comprend un anneau lasik ; soit
la surface de guidage d'aiguille est une première surface de guidage d'aiguille, le canal de guidage d'aiguille est un premier canal de guidage d'aiguille, l'axe d'aiguille est un premier axe d'aiguille, l'ouverture est une première ouverture d'aiguille, et le plan de contact est un premier plan de contact,
dans lequel la surface de contact s'étend au moins partiellement le long d'un second plan de contact, et
dans lequel le boîtier comporte en outre une seconde surface de guidage d'aiguille s'étendant autour d'un second axe de guidage d'aiguille pour définir un second canal de guidage d'aiguille, le second canal de guidage d'aiguille s'étendant à travers le boîtier jusqu'à une seconde ouverture d'aiguille définie par la surface de contact de telle sorte que le second canal de guidage d'aiguille débouche dans la cavité par le biais de la seconde ouverture d'aiguille, l'axe d'aiguille étant sensiblement parallèle au second plan de contact.

11. Guide d'aiguille oculaire selon la revendication 1, conçu pour être positionné sur l'œil et tirer une partie de la sclérotique à l'écart de la choroïde jusque dans la cavité et contre la partie de surface de la surface de contact.

12. Trousse pharmaceutique comprenant :
le guide d'aiguille oculaire selon la revendication 1 ; et
une aiguille (20) conçue pour être insérée à travers le canal de guidage d'aiguille le long de l'axe d'aiguille.

13. Trousse pharmaceutique selon la revendication 12, dans laquelle soit :
une pointe distale de l'aiguille est de forme conique ; soit
une butée (24) est accouplée à l'aiguille, l'aiguille peut être insérée dans la cavité du boîtier jusqu'à une distance prédéterminée, et la butée est positionnée pour empêcher sensiblement l'aiguille de s'étendre jusque dans la cavité sur une distance supérieure à la distance prédéterminée.

14. Guide d'aiguille oculaire (300) pour un accès à un espace sous une sclérotique (14) d'un œil (10), le guide d'aiguille oculaire comprenant :
une première partie de corps (309) ayant une première surface de contact (312, 316) et une surface de guidage d'aiguille (306), la surface de guidage d'aiguille s'étendant autour d'un axe d'aiguille (322) pour définir un canal de guidage d'aiguille (324), le canal de guidage d'aiguille s'étendant à travers le boîtier jusqu'à une ouverture définie par la première surface de contact ; et
une deuxième partie de corps (311) ayant une seconde surface de contact (314), la seconde surface de contact s'étendant au moins partiellement le long d'un plan de contact (320),
la première partie de corps étant mobile par rapport à la seconde partie de corps de telle sorte que le guide d'aiguille oculaire peut effectuer une transition entre une position d'alignement et une position ouverte, dans lequel, dans la position d'alignement, la première surface de contact et la seconde surface de contact définissent une cavité, le canal de guide d'aiguille débouche dans la cavité par le biais de l'ouverture, et l'axe d'aiguille est sensiblement parallèle au plan de contact, et dans lequel, dans la position ouverte, l'axe d'aiguille est décalé angulairement par rapport au plan de contact.

15. Guide d'aiguille oculaire (500) pour un accès à un espace sous une sclérotique (14) d'un œil (10), le guide d'aiguille oculaire comprenant :
un boîtier (502) ayant une première surface de contact (504) et une surface de guidage de montant (505), la première surface de contact définissant une cavité (508) au sein du boîtier, et la surface de guidage de montant définissant un canal de montant (509) au sein du boîtier ; et
un montant (503) positionné au moins partiellement au sein du canal de montant, le montant ayant une seconde surface de contact (509) et une surface de guidage d'aiguille (506), la surface de guidage d'aiguille s'étendant autour d'un axe d'aiguille (522) à travers le montant pour définir un canal de guidage d'aiguille (524), le canal de guidage d'aiguille ayant une ouverture (538) définie par la seconde surface de contact, l'axe d'aiguille ayant une partie d'axe distale (523) qui s'étend de manière sensiblement linéaire depuis un emplacement au sein du canal de guidage d'aiguille à travers l'ouverture, la partie d'axe distale de l'axe d'aiguille étant sensiblement parallèle à au moins une partie de la première surface de contact,
dans lequel le montant est mobile au sein du canal de montant pour définir une première position de profondeur au sein de la cavité et une seconde position de profondeur au sein de la cavité, dans lequel, dans la première position de profondeur, la partie d'axe distale de l'axe d'aiguille est espacée de la partie sensiblement parallèle de la première surface de contact d'une première distance de profondeur, et dans la seconde position de profondeur, la partie d'axe distale de l'axe d'aiguille est espacée de la partie sensiblement parallèle de la première surface de contact d'une seconde distance de profondeur qui est différente de la première distance de profondeur.
